# EUROPEAN PATENT APPLICATION

(11) **EP 1 939 192 A1**
(43) Date of publication of application: **02.07.2008**
(21) Application number: 06127280.3
(22) Date of filing: 28.12.2006
(51) Int. Cl.: C07D 307/58, C07D 307/60, C07D 307/62, C07D 307/66, C07D 333/32, C07D 405/04, C07D 407/04, C07D 407/06, C07F 7/18, A61K 31/341, A61P 25/28, A61P 3/10, A61P 25/24

(54) **Cyclopentanone derivatives, method of synthesis and uses thereof**

(71) Applicant: Neuropharma S.A., 28760 Tres Cantos (Madrid) (ES)
(72) Inventor: Martinez Gil, Ana, 28004 Madrid (ES); Medina Padilla, Miguel, 28035 Madrid (ES); Castro Morera, Ana, 28028 Madrid (ES); Fall Diop, Yagamare, 36208 Vigo - Pontevedra (ES); Gomez Pacios, Generosa, 36208 Vigo - Pontevedra (ES); Pérez Vasquez, Manuel, 36208 Vigo - Pontevedra (ES); Alonso Cascon, Mercedes, 28036 Madrid (ES); Pérez Fernandez, Daniel Igancio, 28034 Madrid (ES); Herrero Santos, Susana, 28921 Alcoron - Madrid (ES); Martin Aparicio, Ester, 28411 Moralzarzal - Madrid (ES); Fuertes Huerta, Ana, 28003 Madrid (ES); Del Monte Millan, Maria, 28035 Madrid (ES); Navarro Rico, Maria Luisa, 28014 Madrid (ES); Pérez Puerto, Maria José, 28008 Madrid (ES)
(74) Representative: ABG Patentes, S.L.

(57) **Abstract**

The present invention relates to cyclopentanone derivatives of formula (I), their method of synthesis and uses thereof. Concretely, the compounds disclosed have proved to be inhibitors of glycogen synthase kinase 3β, GSK-3 β, which is known to be involved in different disease and conditions, such as Alzheimer's disease or non-insulin dependent diabetes mellitus. The present invention also relates to pharmaceutical compositions comprising the same. Further, the present invention is directed to the use of the compounds in the manufacture of a medicament for the treatment and/or prevention of a GSK-3 mediated disease or condition.

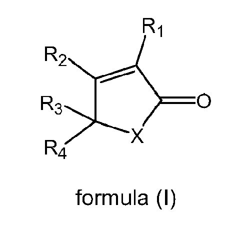

## Description

### FIELD OF THE INVENTION

The present invention relates to cyclopentanone derivatives, their method of synthesis and uses thereof. Concretely, the compounds disclosed have proved to be inhibitors of glycogen synthase kinase 3β, GSK-3 β, which is known to be involved in different diseases and conditions, such as Alzheimer's disease or non-insulin dependent diabetes mellitus. The present invention also relates to pharmaceutical compositions comprising the same. Further, the present invention is directed to the use of the compounds in the manufacture of a medicament for the treatment and/or prevention of a GSK-3 mediated disease or condition.

### BACKGROUND OF THE INVENTION

The search for new therapeutic agents has been greatly aided in recent years by better understanding the structure of enzymes and other biomolecules associated with target diseases. One important class of enzymes that has been the subject of extensive study is the protein kinases. Many diseases are associated with abnormal cellular responses triggered by protein kinase-mediated events. These diseases include autoimmune diseases, inflammatory diseases, neurological and neurodegenerative diseases, cancer, cardiovascular diseases, allergies and asthma, Alzheimer's disease or hormone-related diseases. Accordingly, there has been a substantial effort in medicinal chemistry to find protein kinase inhibitors that are effective as therapeutic agents.

Glycogen synthase kinase-3 (GSK-3) is a serine/threonine protein kinase comprised of α and β isoforms that are each encoded by distinct genes (Coghlan et al., Chemistry & Biology, 7, 793-803 (2000); Kim and Kimmel, Curr. Opinion Genetics Dev., 10, 508-514 (2000)). The threonine/serine kinase glycogen synthase kinase-3 (GSK-3) fulfills a pivotal role in various receptor-linked signalling pathways (Doble, BW, Woodgett, JR J.Cell Sci. 2003, 116:1175-1186). Dysregulation within these pathways is considered a crucial event in the development of several prevalent human disorders, such as type II diabetes (Kaidanovich O, Eldar-Finkelman H, Expert Opin. Ther. Targets, 2002, 6:555-561), Alzheimer's disease (Grimes CA, Jope RS, Prog.Neurobiol. 2001, 65:391-426), CNS disorders such as manic depressive disorder and neurodegenerative diseases, and chronic inflammatory disorders (Hoeflich KP, Luo J, Rubie EA, Tsao MS, Jin O, Woodgett J, Nature 2000, 406:86-90). These diseases may be caused by, or result in, the abnormal operation of certain cell signalling pathways in which GSK-3 plays a role.

GSK-3 has been found to phosphorylate and modulate the activity of a number of regulatory proteins. These proteins include glycogen synthase which is the rate limiting enzyme necessary for glycogen synthesis, the microtubule associated protein Tau, the gene transcription factor β-catenin, the translation initiation factor e1 F2B, as well as ATP citrate lyase, axin, heat shock factor-1, c-Jun, c-Myc, c-Myb, CREB, and CEPBα. These diverse protein targets implicate GSK-3 in many aspects of cellular metabolism, proliferation, differentiation and development.

Currently, inhibition of GSK-3 may represent a viable strategy to develop novel medicinal entities for the treatment of such unmet diseases (Martinez A, Castro A, Dorronsoro I, Alonso M, Med. Res. Rev., 2002, 22:373-384) through insulin mimicry, tau dephosphorylation and amyloid processing, or transcriptional modulation respectively.

There is still a need to find good GSK-3 inhibitors, being both effective and selective, and having good "drugability" properties, i.e. good pharmaceutical properties related to administration, distribution, metabolism and excretion. An additional advantage would be to find compounds with simple, stable structures, being easy to prepare by ordinary proceedings known to the skilled person.

### SUMMARY OF THE INVENTION

It has now been found that a group of stable and small cyclopentanone derivatives shows inhibitory effects on GSK-3 enzyme.

Thus, according to a first aspect, the present invention is directed to a compound of formula (I) (herein referred to as "compounds of the invention") 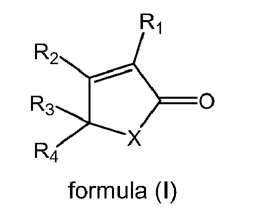 wherein
R₁ is selected from the group consisting of -H, -ORₐ, alkyl and aralkyl, wherein
Rₐ is selected from the group consisting of-H and hydroxyl protecting groups;
R₂ is selected from the group consisting of-H, -ORₐ, alkyl and -NR_{b}, wherein
Rₐ is as defined above; and
R_{b} together with the nitrogen atom forms an optionally substituted heterocyclyl group;
R₃ is -H or -O-Alkyl;
R₄ is selected from the group consisting of -H, alkyl, alkenyl, aryl, aralkyl and heterocyclyl, wherein the alkyl and the alkenyl groups are optionally substituted by at least one -ORₐ group, wherein -ORₐ has the same meaning as above;
X is an oxygen or sulphur atom; and
wherein at least two of R₁, R₂, R₃ and R₄ are not -H;
and its salts or solvates or tautomers thereof.

According to a further aspect, the present invention is directed to a pharmaceutical composition comprising at least one compound of formula (I) as defined above, its salts or solvates or tautomers thereof, and at least one pharmaceutically acceptable carrier.

According to a further aspect, the present invention is directed to a compound of formula (I) as defined above, its salts, solvates or tautomers thereof, for use as a medicament.

According to a further aspect, the present invention is directed to the use of at least one compound of formula (I) as defined above, its salts or solvates or tautomers thereof, as reagent for biological assays, preferably as a reagent for GSK-3 inhibition.

According to a further aspect, the present invention is directed to the use of a compound of formula (I) 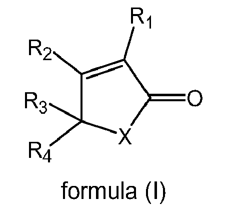 wherein
R₁ is selected from the group consisting of -H, -ORₐ, alkyl and aralkyl, wherein
Rₐ is selected from the group consisting of-H and hydroxyl protecting groups;
R₂ is selected from the group consisting of-H, -ORₐ, alkyl and -NR_{b}, wherein
Rₐ is as defined above; and
R_{b} together with the nitrogen atom forms an optionally substituted heterocyclyl group;
R₃ is -H or -O-Alkyl;
R₄ is selected from the group consisting of -H, aryl, aralkyl, heterocyclyl, alkyl and alkenyl, said alkyl or alkenyl group being optionally substituted by at least one heterocycle, preferably furane; and optionally at least one -ORₐ group, wherein -ORₐ has the same meaning as above; or
R₃ and R₄ together form an alkenyl group attached to the lactone ring by a double bond, optionally substituted by at least one heterocycle, preferably furane;
X is an oxygen or sulphur atom; and
wherein at least two of R₁, R₂, R₃ and R₄ are not -H;
and its salts or solvates or tautomers thereof;
in the manufacture of a medicament for the treatment and/or prevention of a GSK-3 mediated disease or condition.

According to a further aspect, the present invention is directed to a method of treating or preventing a GSK-3 mediated disease or condition, which method comprises administering to a patient in need of such a treatment a therapeutically effective amount of at least one compound of formula (I) as defined above, its salts, solvates or tautomers thereof, or a pharmaceutical composition thereof.

According to a further aspect, the present invention is directed to a process for the synthesis of the compounds of formula I, its salts or solvates or tautomers thereof.

### DETAILED DESCRIPTION OF THE INVENTION

### Compounds of formula I

As mentioned above, an aspect of the present invention is a compound of formula I, its salts or solvates or tautomers thereof.

As mentioned above, the compounds of the invention also encompass their tautomers. The skilled person is aware that a carbonyl functionality may be present as an equilibrium between two tautomeric forms: the enol form and the keto form. Enols are usually unstable and the equilibrium between both is usually displaced towards the keto form as shown in scheme 1:

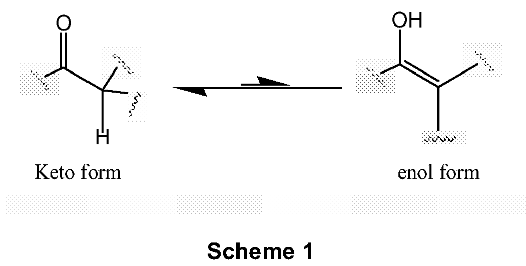

It is known in the art that the extent of enolization depends *inter alia* on the nature of the compound, the solvent, the concentration and the temperature (Toullec, in Rappoport, Ref. 264a., pages 323-398). In molecules where there is an unsaturated β-hydroxycarbonyl (or β-diketones) moiety having the formula, 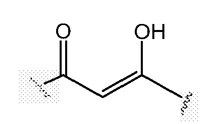 there are two possible tautomeric enol forms as shown in scheme 2:

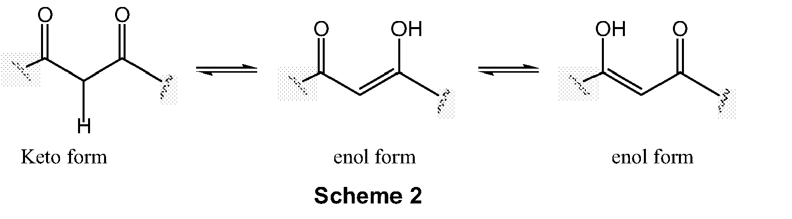

The conjugation between the enolic double bond and the ketone double bond stabilizes the enol form. For example, the enol content of CH₃C(=O)OCH₂CH₃ is cero, while the enol content of CH₃(C=O)CH₂C(=O)OCH₂CH₃ is 8.4%. Further conjugation can turn the enol form into the predominant tautomer. For example, the enol content of Ph(C=O)CH₂C(=O)CH₃ is 89.2 %.

It is also known in the art that esters or thioesters tend to have a smaller amount of the enol form than, for example, ketones or aldehydes. For example, the enol content of CH₃C(=O)OCH₂CH₃ is cero, while the enol content of CH₃CHO is 6·10⁻⁵.

Thus, the compounds of the invention wherein R₂ is -OH can be equally represented as the keto form, the enol form A or the enol form B shown in scheme 3:

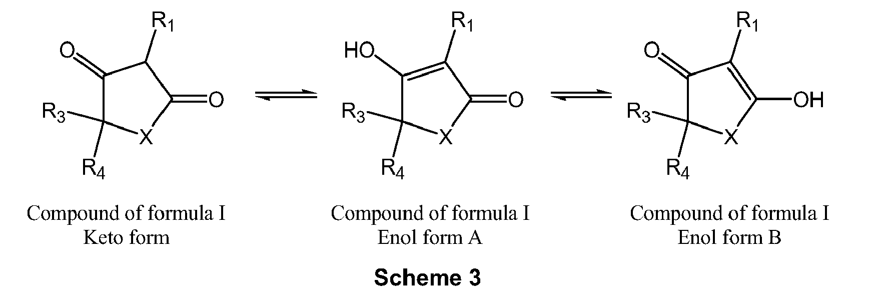

Thus, depending on the conditions and their structure, the compounds of formula (I) may be present as single tautomer (e.g. the keto form, the enol form A or the enol form B) or as an equilibrium of two or all three of tautomeric forms (e.g. an equilibrium between the keto form and the enol form A; or an equilibrium between the keto form and the enol form B; or an equilibrium between the enol form A and the enol form B; or an equilibrium between the keto form, the enol form A and the enol form B).

The compounds of formula (I) may be in the form of salts, preferably pharmaceutically acceptable salts, or in the form of solvates. The term **"pharmaceutically acceptable salts"** refers to any salt which upon administration to the recipient is capable of providing (directly or indirectly) a compound as described herein. However, it will be appreciated that non-pharmaceutically acceptable salts also fall within the scope of the invention since those may be useful in the preparation of pharmaceutically acceptable salts. Preferably, "pharmaceutically acceptable" refers to molecular entities and compositions that are physiologically tolerable and do not typically produce an allergic or similar untoward reaction, such as gastric upset, dizziness and the like, when administered to a human. Preferably, as used herein, the term "pharmaceutically acceptable" means approved by a regulatory agency of the Federal or a state government or listed in the U.S. Pharmacopeia or other generally recognized pharmacopeia for use in animals, and more particularly in humans.

The term **"solvate"** according to this invention is to be understood as meaning any form of the active compound according to the invention which has another molecule (most likely a polar solvent) attached to it via non-covalent bonding. Examples of solvates include hydrates and alcoholates, e.g. methanolate. Preferably, the solvates are pharmaceutically acceptable solvates.

The preparation of salts and solvates can be carried out by methods known in the art. For instance, pharmaceutically acceptable salts of compounds provided herein are synthesized from the parent compound which contains a basic moiety by conventional chemical methods. Generally, such salts are, for example, prepared by reacting the free base forms of these compounds with a stoichiometric amount of the appropriate base or acid in water or in an organic solvent or in a mixture of the two. Generally, non-aqueous media like ether, ethyl acetate, ethanol, isopropanol or acetonitrile are preferred. Examples of the acid addition salts include mineral acid addition salts such as, for example, hydrochloride, hydrobromide, hydroiodide, sulphate, nitrate, phosphate, and organic acid addition salts such as, for example, acetate, maleate, fumarate, citrate, oxalate, succinate, tartrate, malate, mandelate, methanesulphonate and p-toluenesulphonate.

One preferred pharmaceutically acceptable form is the crystalline form, including such form in a pharmaceutical composition. In the case of salts and solvates the additional ionic and solvent moieties must also be non-toxic. The compounds of the invention may present different **polymorphic forms**, it is intended that the invention encompasses all such forms.

According to an embodiment of the invention, the compound of formula (I) is a compound of formula (II) 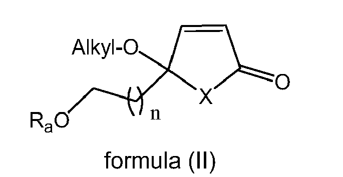 wherein
X is an oxygen or sulphur atom;
n is an integer selected from 1, 2, 3, 4, 5 and 6; and
Rₐ is as defined above in formula (I);
and its salts or solvates thereof.

According to a preferred embodiment, Rₐ in the compound of formula (II) is a hydroxyl protecting group, preferably a silylether.

According to an embodiment of the invention, the compound of formula (I) is a compound of formula (IV) 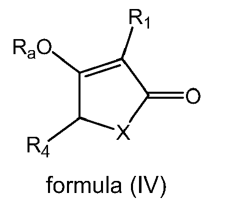 wherein
X is an oxygen or sulphur atom;
R₁, R₄ and Rₐ are as defined above in formula (I);
and its salts or solvates or tautomers thereof.

According to a preferred embodiment, R₁ is methyl in a compound of formula (IV).

According to a further embodiment, R₄ in a compound of formula (IV) is an alkenyl of formula (VII) 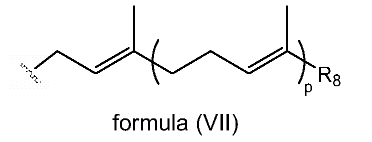 wherein
at least one double bond may be optionally replaced by an epoxide;
p is an integer selected from 0, 1, 2 or 3; and
R₈ is methyl or-ORₐ, wherein Rₐ is as defined above in formula (I);
and its salts or solvates or tautomers thereof.

The moieties of formula (VII) comprise at least one double bond, which may be optionally substituted by an epoxide ring, for example, moieties having the following formulas:

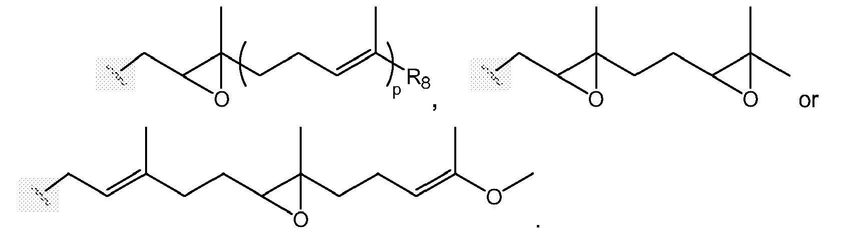

There are many methods for the epoxidation of double bonds known in the art. Reference is made to pages 826-829 of March, J. "Advanced Organic Chemistry: Reactions , mechanisms, and structure", forth edition, Ed.: Wiley-Interscience.

According to an embodiment of the invention, the compound of formula (I) is a compound of formula (V) 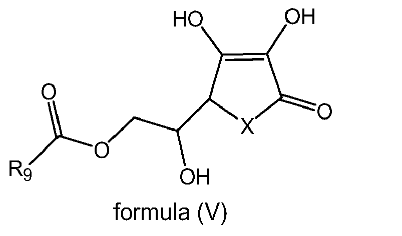 wherein
X is an oxygen or sulphur atom; and
R₉ is selected from the group consisting of -H, alkyl, alkenyl, aryl, aralkyl and heterocyclyl;
and its salts or solvates or tautomers thereof.

According to an embodiment of the invention, the compound of formula (I) is a compound of formula (VI) 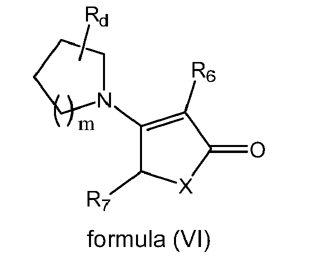 wherein
X is an oxygen or sulphur atom;
m is an integer selected from 1, 2 and 3;
R₆ is alkyl group;
R₇ is alkyl or alkenyl group; and
R_{d} is selected from the group consisting of hydrogen, substituted or unsubstituted alkyl, substituted or unsubstituted alkenyl, substituted or unsubstituted arylalkyl and substituted or unsubstituted alkoxy.
and its salts or solvates or tautomers thereof.

According to a preferred embodiment, R₇ in a compound of formula (V)I is an alkenyl of formula (VII) 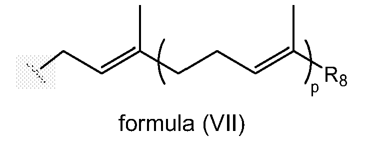 wherein
at least one double bond may be optionally replaced by an epoxide;
p is an integer selected from 0, 1, 2 and 3; and
R₈ is methyl or -ORₐ, wherein Rₐ is as defined above in formula (I).

As mentioned above, the compounds of formula (I) wherein R₂ is -OH may be equally represented by three tautomeric forms (see scheme 3): keto form, enol form A and enol form B. Thus, the compounds of formula (I) wherein R₂ is -OH may have two reactive oxygen atoms as shown is scheme 4.

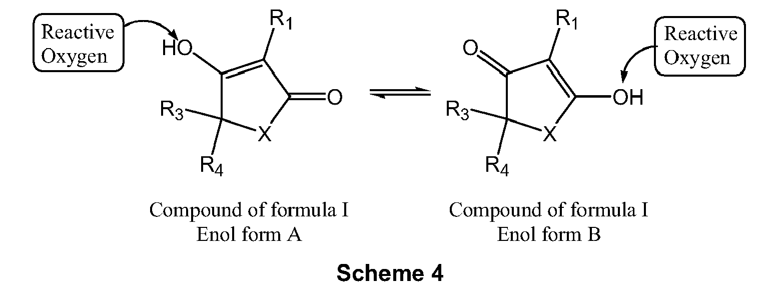

The skilled person is aware that enols may be trapped by reaction of the reactive enolic oxygen with an electrophile. Typical examples of such trapped enols are sylilenol ether, alkylenol ethers, arylenol ethers or aralkylenol ethers.

For example, the compounds of the invention wherein R₂ is -ORₐ, Rₐ being different from hydrogen, represent enol form A of the compound of formula (I,) wherein said enol form A has been trapped by a suitable electrophile.

Alternatively, compounds of formula (I) wherein enol form B is the mayor tautomer may be trapped by a suitable electrophile as described above and be in the form of the corresponding silylenol ether, alkylenol ether, arylenol ether or an aralkylenol ether.

Thus, according to a further embodiment, the present invention is directed to a compound of formula (VIII) 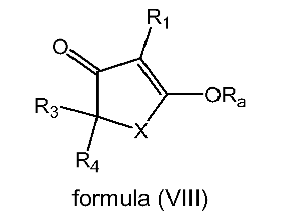 wherein X, R₁, R₃ and R₄ are defined as above in formula (I); and
Rₐ is a hydroxyl protecting group;
and its salts or solvates thereof.

According to a preferred embodiment, Rₐ in said compound of formula (VIII) is an alkyl group.

According to a preferred embodiment, the compound of formula (I) is selected from the group consisting of
1. 5-[2-(tert-Butyl-diphenyl-silanyloxy)-ethyl]-5-methoxy-5*H*-furan-2-one
2. 5-[3-(tert-Butyl-diphenyl-silanyloxy)-propyl]-5-methoxy-5*H*-furan-2-one
3. 5-[4-(tert-Butyl-diphenyl-silanyloxy)-butyl]-5-methoxy-5H-furan-2-one
4. 5-(4-hydroxybutyl)-5-methoxy-5H-furan-2-one
5. 5-[5-(tert-Butyl-diphenyl-silanyloxy)-pentyl]-5-methoxy-5H-furan-2-one
6. 5-[6-(tert-Butyl-diphenyl-silanyloxy)-hexyl]-5-methoxy-5H-furan-2-one
7. 5-[4-(tert-Butyl-diphenyl-silanyloxy)-3-methyl-butyl]-4-hydroxy-3-methyl-5H-furan-2-one
8. 5-[7-(tert-Butyl-diphenyl-silanyloxy)-2,6-dimethyl-hept-2-enyl]-4-methoxy-3-methyl-5H-furan-2-one
9. 5-(6-Furan-3-yl-3-methyl-hex-2-enyl)-4-methoxy-3-methyl-5H-furan-2-one
10. 4-Hydroxy-3-naphthalen-2-ylmethyl-5H-furan-2-one
11. 3-Benzyl-4-hydroxy-5H-furan-2-one
12. 4-Hydroxy-3-methyl-5H-thiophen-2-one
13. 4-Hydroxy-3-pentyl-5H-furan-2-one
14. 4-Hydroxy-3-methyl-5-(3,7,11-trimethyl-dodeca-2,6,10-trienyl)-5H-furan-2-one
15. 4-Hydroxy-3-methyl-5-(3,7,11-trimethyl-dodeca-2,6,10-trienyl)-5H-thiophen-2-one
16. 5-(3,7-Dimethyl-octa-2,6-dienyl)-4-hydroxy-3-methyl-5H-furan-2-one
17. 4-Hydroxy-3-methyl-5-undec-1 0-enyl-5H-furan-2-one
18. [4-(3-Hydroxy-4-methyl-5-oxo-2,5-dihydro-furan-2-ylmethyl)-phenyl]-acetic acid 2-oxo-2-phenyl-ethyl ester
19. 4-Methoxymethoxy-3-methyl-5-(3,7,11-trimethyl-dodeca-2,6,10-trienyl)-5H-furan-2-one
20. 5-(4-Hydroxy-3-methyl-but-2-enyl)-4-methoxymethoxy-3-methyl-5H-furan-2-one
21. Acetic acid 4-methyl-5-oxo-2-(3,7,11-trimethyl-dodeca-2,6,10-trienyl)-2,5-dihydro-furan-3-yl ester
22. Acetic acid 4-methyl-5-oxo-2-(3,7,11-trimethyl-dodeca-2,6,10-trienyl)-2,5-dihydro-furan-3-yl ester
23. Acetic acid 4-methyl-5-oxo-2-undec-10-enyl-2,5-dihydro-furan-3-yl ester
24. Acetic acid 4-methyl-5-oxo-2-(3,7,11-trimethyl-dodeca-2,6,10-trienyl)-2,5-dihydro-thiophen-3-yl ester
25. 4-Methoxymethoxy-3-methyl-5-{3-methyl-5-[3-methyl-3-(4-methyl-pent-3-enyl)-oxiranyl]-pent-2-enyl}-5*H*-furan-2-one
26. 5-[9-(3,3-Dimethyl-oxiranyl)-3,7-dimethyl-nona-2,6-dienyl]-4-methoxymethoxy-3-methyl-5H-furan-2-one
27. 4-Methoxy-3-methyl-5-(3,7,11-trimethyl-dodeca-2,6,10-trienyl)-5H-furan-2-one
28. 3-Methyl-4-propoxy-5-(3,7,11-trimethyl-dodeca-2,6,10-trienyl)-5*H*-furan-2-one
29. Toluene-4-sulfonic acid 4-methyl-5-oxo-2,5-dihydro-furan-3-yl ester
30. 5-(3,7-Dimethyl-octa-2,6-dienyl)-4-methoxymethoxy-3-methyl-5*H*-furan-2-one
31. 5-(2,2-Dimethyl-[1,3]dioxolan-4-yl)-3,4-dihydroxy-5H-furan-2-one
32. Hexadecanoic acid 2-(3,4-dihydroxy-5-oxo-2,5-dihydro-furan-2-yl)-2-hydroxy-ethyl ester
33. 3-Phenyl-acrylic acid 2-(3,4-dihydroxy-5-oxo-2,5-dihydro-furan-2-yl)-2-hydroxy-ethyl ester
34. Decanoic acid 2-(3,4-dihydroxy-5-oxo-2,5-dihydro-furan-2-yl)-2-hydroxy-ethyl ester
35. But-2-enoic acid 2-(3,4-dihydroxy-5-oxo-2,5-dihydro-furan-2-yl)-2-hydroxy-ethyl ester
36. Hexanedioic acid 2-(3,4-dihydroxy-5-oxo-2,5-dihydro-furan-2-yl)-2-hydroxy-ethyl ester vinyl ester
37. Undec-10-enoic acid 2-(3,4-dihydroxy-5-oxo-2,5-dihydro-furan-2-yl)-2-hydroxy-ethyl ester
38. Dodecanoic acid 2-(3,4-dihydroxy-5-oxo-2,5-dihydro-furan-2-yl)-2-hydroxy-ethyl ester
39. Octanoic acid 2-(3,4-dihydroxy-5-oxo-2,5-dihydro-furan-2-yl)-2-hydroxy-ethyl ester
40. Hexa-2,4-dienoic acid 2-(3,4-dihydroxy-5-oxo-2,5-dihydro-furan-2-yl)-2-hydroxy-ethyl ester
41. Chloro-acetic acid 2-(3,4-dihydroxy-5-oxo-2,5-dihydro-furan-2-yl)-2-hydroxy-ethyl ester
42. Butyric acid 2-(3,4-dihydroxy-5-oxo-2,5-dihydro-furan-2-yl)-2-hydroxy-ethyl ester
43. Tetradecanoic acid 2-(3,4-dihydroxy-5-oxo-2,5-dihydro-furan-2-yl)-2-hydroxy-ethyl ester
44. Octadecanoic acid 2-(3,4-dihydroxy-5-oxo-2,5-dihydro-furan-2-yl)-2-hydroxy-ethyl ester
45. 5-(13-Furan-3-yl-2,6,10-trimethyl-trideca-2,7,9-trienyl)-4-hydroxy-3-methyl-5*H-*furan-2-one
46. 3-Methyl-4-pyrrolidin-1-yl-5-(3,7,11-trimethyl-dodeca-2,6,10-trienyl)-5*H*-furan-2-one
47. 4-Methyl-5-propoxy-2-(3,7,11-trimethyl-dodeca-2,6,10-trienyl)-furan-3-one
48. 6-(4-Methyl-5-oxo-3-pyrrolidin-1-yl-2,5-dihydro-furan-2-yl)-hexanenitrile
49. 4-(2-Methoxymethyl-pyrrolidin-1-yl)-3-methyl-5-(3,7,11-trimethyl-dodeca-2,6,10-trienyl)-5H-furan-2-one
50. 4-(2-Methoxymethyl-pyrrolidin-1-yl)-3-methyl-5-(3,7,11-trimethyl-dodeca-2,6,10-trienyl)-5H-furan-2-one
51. 4-sec-Butoxy-3-methyl-5-(3,7,11-trimethyl-dodeca-2,6,10-trienyl)-5H-furan-2-one
52. 5-sec-Butoxy-4-methyl-2-(3,7,11-trimethyl-dodeca-2,6,10-trienyl)-furan-3-one
53. 5-(12-Hydroxy-3,7,11-trimethyl-dodeca-2,6,10-trienyl)-3-methyl-4-pyrrolidin-1-yl-5H-furan-2-one
its salts or solvates or tautomers thereof. The numbering given to the compounds above is the number given in the examples regarding biological activity

### Pharmaceutical compositions

According to a further aspect, the present invention is directed to a pharmaceutical composition comprising at least one compound of formula (I) as defined above, its salts or solvates or tautomers thereof, and at least one pharmaceutically acceptable carrier.

The term "**carrier**" refers to a **diluent, adjuvant, excipient, or vehicle** with which the active ingredient is administered. Such pharmaceutical carriers can be sterile liquids, such as water and oils, including those of petroleum, animal, vegetable or synthetic origin, such as peanut oil, soybean oil, mineral oil, sesame oil and the like. Water or aqueous solution saline solutions and aqueous dextrose and glycerol solutions are preferably employed as carriers, particularly for injectable solutions. Suitable pharmaceutical carriers are described in "Remington's Pharmaceutical Sciences" by E.W. Martin, 1995.

Preferably, the carriers of the invention are approved by a regulatory agency of the Federal or a state government or listed in the U.S. Pharmacopeia or other generally recognized pharmacopeia for use in animals, and more particularly in humans

The carriers and auxiliary substances necessary to manufacture the desired pharmaceutical form of administration of the pharmaceutical composition of the invention will depend, among other factors, on the elected administration pharmaceutical form. Said pharmaceutical forms of administration of the pharmaceutical composition will be manufactured according to conventional methods known by the skilled person in the art. A review of different active ingredient administration methods, excipients to be used and processes for producing them can be found in "Tratado de Farmacia Galenica", C. Faulí i Trillo, Luzán 5, S.A. de Ediciones, 1993.

Examples of pharmaceutical compositions include any solid (tablets, pills, capsules, granules etc.) or liquid (solutions, suspensions or emulsions) composition for oral, topical or parenteral administration.

In a preferred embodiment the pharmaceutical compositions are in oral form. Suitable dose forms for oral administration may be tablets and capsules and may contain conventional excipients known in the art such as binding agents, for example syrup, acacia, gelatin, sorbitol, tragacanth, or polyvinylpyrrolidone; fillers, for example lactose, sugar, maize starch, calcium phosphate, sorbitol or glycine; tabletting lubricants, for example magnesium stearate; disintegrants, for example starch, polyvinylpyrrolidone, sodium starch glycolate or microcrystalline cellulose; or pharmaceutically acceptable wetting agents such as sodium lauryl sulfate.

The solid oral compositions may be prepared by conventional methods of blending, filling or tabletting. Repeated blending operations may be used to distribute the active agent throughout those compositions employing large quantities of fillers. Such operations are conventional in the art. The tablets may for example be prepared by wet or dry granulation and optionally coated according to methods well known in normal pharmaceutical practice, in particular with an enteric coating.

The pharmaceutical compositions may also be adapted for parenteral administration, such as sterile solutions, suspensions or lyophilized products in the appropriate unit dosage form. Adequate excipients can be used, such as bulking agents, buffering agents or surfactants.

The mentioned formulations will be prepared using standard methods such as those described or referred to in the Spanish and US Pharmacopoeias and similar reference texts.

Administration of the compounds or compositions of the present invention may be by any suitable method, such as intravenous infusion, oral preparations, and intraperitoneal and intravenous administration. Oral administration is preferred because of the convenience for the patient and the chronic character of many of the diseases to be treated.

Generally an effective administered amount of a compound of the invention will depend on the relative efficacy of the compound chosen, the severity of the disorder being treated and the weight of the sufferer. However, active compounds will typically be administered once or more times a day for example 1, 2, 3 or 4 times daily, with typical total daily doses in the range of from 0.01 to 1000 mg/kg/day.

According to a further aspect, the present invention is directed to a compound of formula (I), its salts or solvates or tautomers thereof, as defined above for use as a medicament.

According to a further aspect, the present invention is directed to a method of treating or preventing a GSK-3 mediated disease or condition, which method comprises administering to a patient in need of such a treatment a therapeutically effective amount of at least one compound of formula (I), its salts or solvates or tautomers thereof, as defined above or a pharmaceutical composition thereof.

The term "treatment" or "to treat" in the context of this specification means administration of a compound or formulation according to the invention to prevent, ameliorate or eliminate the disease or one or more symptoms associated with said disease. "Treatment" also encompasses preventing, ameliorating or eliminating the physiological sequelae of the disease.

The term "ameliorate" in the context of this invention is understood as meaning any improvement on the situation of the patient treated - either subjectively (feeling of or on the patient) or objectively (measured parameters).

According to a further aspect, the present invention is directed to the use of a compound of formula (I), its salts or solvates or tautomers thereof, as defined above as reagents for biological assays, preferably as a reactive for GSK-3 inhibition. Said method comprises contacting a biological sample with a GSK-3 inhibitor of formula I. The term **"biological sample",** as used herein, includes, without limitation, cell cultures or extracts thereof; preparations of an enzyme suitable for in vitro assay; biopsied material obtained from a mammal or extracts thereof; and blood, saliva, urine, feces, semen, tears, or other body fluids or extracts thereof.

### Uses of compounds of formula (I) and pharmaceutical compositions

As mentioned above, a further aspect of the present invention is the use of a compound of formula (I) 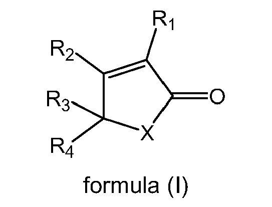 wherein
R₁ is selected from the group consisting of -H, -ORₐ, alkyl and aralkyl, wherein
Rₐ is selected from the group consisting of -H and hydroxyl protecting groups;
R₂ is selected from the group consisting of -H, -ORₐ, alkyl and -NR_{b}, wherein
Rₐ is as defined above; and
R_{b} together with the nitrogen atom forms an optionally substituted heterocyclyl group;
R₃ is -H or -O-Alkyl;
R₄ is selected from the group consisting of -H, aryl, aralkyl, heterocyclyl, alkyl and alkenyl, said alkyl or alkenyl group being optionally substituted by at least one heterocycle, preferably furane; and optionally at least one -ORa group, wherein -ORa has the same meaning as above; or
R₃ and R₄ together form an alkenyl group attached to the lactone ring by a double bond, optionally substituted by at least one heterocycle, preferably furane;
X is an oxygen or sulphur atom; and
wherein at least two of R₁, R₂, R₃ and R₄ are not -H;
and its salts or solvates or tautomers thereof;
in the manufacture of a medicament for the treatment and/or prevention of a GSK-3 mediated disease or condition.

Within the present invention, the expression "GSK-3 mediated disease or condition" means any disease or other deleterious condition or state in which GSK-3 is known to play a role. According to a preferred embodiment, said GSK-3 mediated disease or condition is selected from diabetes, conditions associated with diabetes, chronic neurodegenerative conditions including dementias such as Alzheimer's Disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, corticobasal degeneration, frontotemporal dementia, Huntington's Disease, AIDS associated dementia, amyotrophic lateral sclerosis, multiple sclerosis and neurotraumatic diseases such as acute stroke, epilepsy, mood disorders such as depression, schizophrenia and bipolar disorders, promotion of functional recovery post stroke, cerebral bleeding such as due to solitary cerebral amyloid angiopathy, hair loss, obesity, atherosclerotic cardiovascular disease, hypertension, polycystic ovary syndrome, syndrome X, ischaemia, brain injury, traumatic brain injury, cancer, leukopenia, Down's syndrome, Lewy body disease, inflammation, chronic inflammatory diseases, cancer and hyperproliferative diseases as hyperplasias and immunodeficiency.

According to a preferred embodiment, said GSK-3 mediated disease or condition is Alzheimer's Disease.

According to a preferred embodiment, said GSK-3 mediated disease or condition is type II diabetes.

According to a preferred embodiment, said GSK-3 mediated disease or condition is depression.

According to a preferred embodiment, said GSK-3 mediated disease or condition is brain injury.

According to an embodiment of the present invention, said compound of formula (I) is selected from the group consisting of 5-[9-(5-Furan-3-ylmethyl-furan-3-yl)-2,6-dimethyl-non-6-enylidene]-4-hydroxy-3-methyl-5H-furan-2-one, 5-[9-(5-Furan-3-ylmethyl-furan-3-yl)-2,6-dimethyl-non-5-enylidene]-4-hydroxy-3-methyl-5H-furan-2-one and 5-(13-Furan-3-yl-2,6,10-trimethyl-trideca-6,10-dienylidene)-4-hydroxy-3-methyl-5H-furan-2-one; or its salts, solvates or tautomers thereof.

The compounds and compositions of this invention may be used with other drugs to provide a combination therapy. The other drugs may form part of the same composition, or be provided as a separate composition for administration at the same time or at different time.

### Process for the synthesis of a compound of formula I

The compounds of the present invention may be prepared by a combination of reactions known in the art.

For example, a starting material for the synthesis of a compound of formula (I) may be a substituted or unsubstituted furan or thiophene (scheme 5). Alkylation of a substituted or unsubstituted furan or thiophene with an alkyl halide of formula R₄-Hal, wherein R₄ is as defined in formula (I) and Hal is a halogen atom, provides the 2-substituted derivative as mayor product. Further radical oxidation in the presence of an alcohol of formula Alkyl-OH, provides a hydroperoxide which may be further transformed into a compound of formula (XIII) by treatment with base and anhydride:

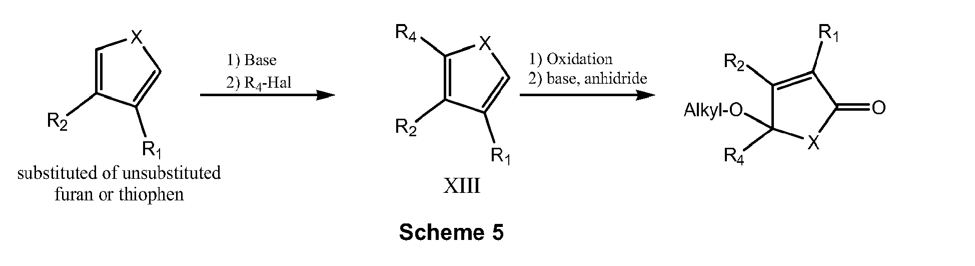

This strategy is especially advantageous for the synthesis of compounds of formula (II), wherein R₁ and R₂ are hydrogen atoms (scheme 6):

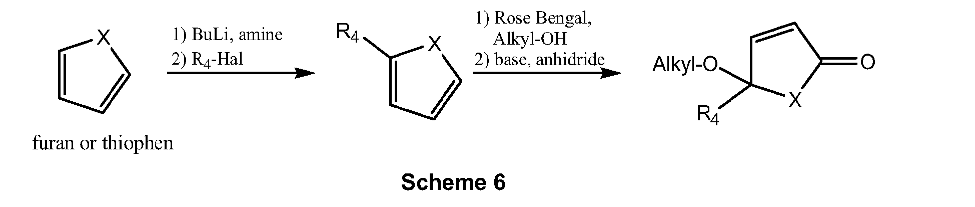

A further strategy for the synthesis of compounds of formula (I) is based on the direct synthesis of the partially substituted or unsubstituted 5*H*-furan-2-one or 5H-thiophen-2-one rings.

For example, 5*H*-thiophen-2-ones may be obtained by cyclation of a compound of formula (IX) in the presence of a base 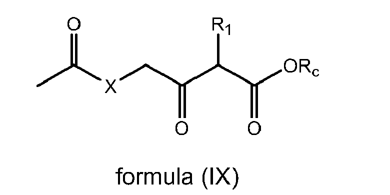 wherein R₁ and X are as defined above in formula (I) and OR_{c} forms an ester group, preferably R_{c} being selected from the group comprising substituted or unsubstituted alkyl, substituted or unsubstituted cycloalkyl, substituted or unsubstituted alkenyl, substituted or unsubstituted cycloalkenyl, substituted or unsubstituted heterocyclyl, substituted or unsubstituted aryl and substituted or unsubstituted arylalkyl.

Said compound of formula (IX) is readily available by bromination of a compound of formula (XI) 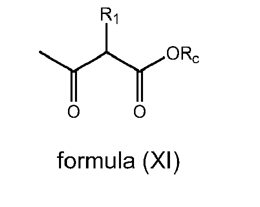 wherein R₁ is as defined in formula (I) and R_{c} is as defined above, followed by addition of a base and a thiolalkyl acid of formula R_{c}(C=O)SH.

5*H*-furan-2-one may be obtained, for example, in a one-pot reaction comprising the bromination of the above mentioned compound of formula (XI) followed by stirring under heat.

This strategy for the synthesis of 5*H*-furan-2-ones or 5*H*-thiophen-2-ones readily provides compounds of formula (IV) wherein the Rₐ is a hydrogen atom (scheme 7):

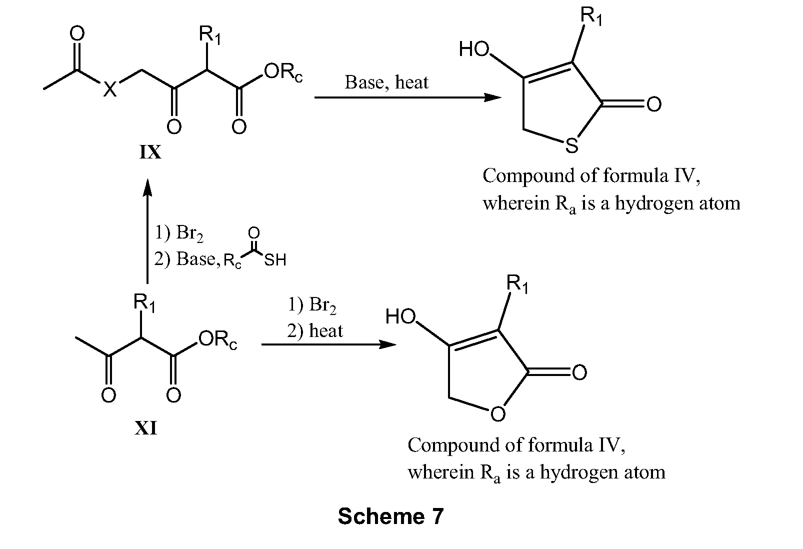

Transformation of the compounds obtained in this way may provide further compounds of formula (IV) and also compounds of formula (VI) and (VIII). For example, alkylation of a compound of formula (XII) 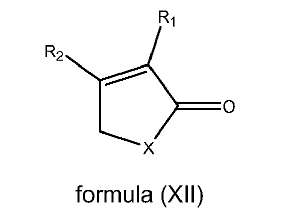 wherein R₁, R₂ and X are as defined in formula (I), in the presence of compound of formula R₄-Hal, wherein R₄ is as defined in formula (I) and Hal is a halogen atom, provides 5-substituted 5*H*-furan-2-ones or 5*H*-thiophen-2-ones of formula (XIV): 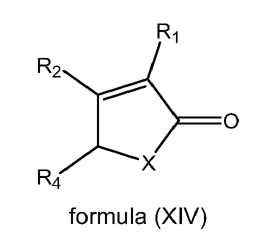 wherein R₁, R₂, R₄ and X are as defined in formula (I).

When the compound of the invention is a compound of formula (IV) wherein R₂ is OH, it is possible to further introduce a protecting group so as to obtain a compound of formula (IV) wherein R₂ is -ORₐ. Examples of such transformations are well known to the skilled person. Comprehensive examples can be found below (see examples 9 and 16) and on pages 24 to 299 of Greene and Wuts' "Protective Groups in Organic Synthesis", John Wiley & Sons, Inc., New York, 4th Ed., 2007.

Alternatively, treatment with an alcohol, triphenylphosphine and diisopropyl azodicarboxylate (DIAD) traps the tautomer of the compound of formula (IV) as an enol ether of formula (VIII).

Also, it is possible to obtain compounds of formula (V) by treatment of compounds of formula (IV) wherein R₂ is -OH with a cyclic amine compound of formula (XV): 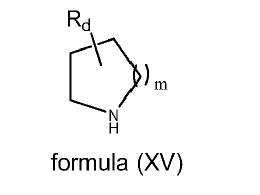 wherein m and R_{d} are as defined in formula (V).

The order of the reactions described above depends on the nature of the specific substituents, and different combinations of the above mentioned reactions are possible. For example, it is possible to first obtain a compound of formula (V) with substitution in position 5 of the 5*H*-furan-2-one or 5*H*-thiophen-2-one ring and then perform an alkylation in position 5 or vice versa (see scheme 8)

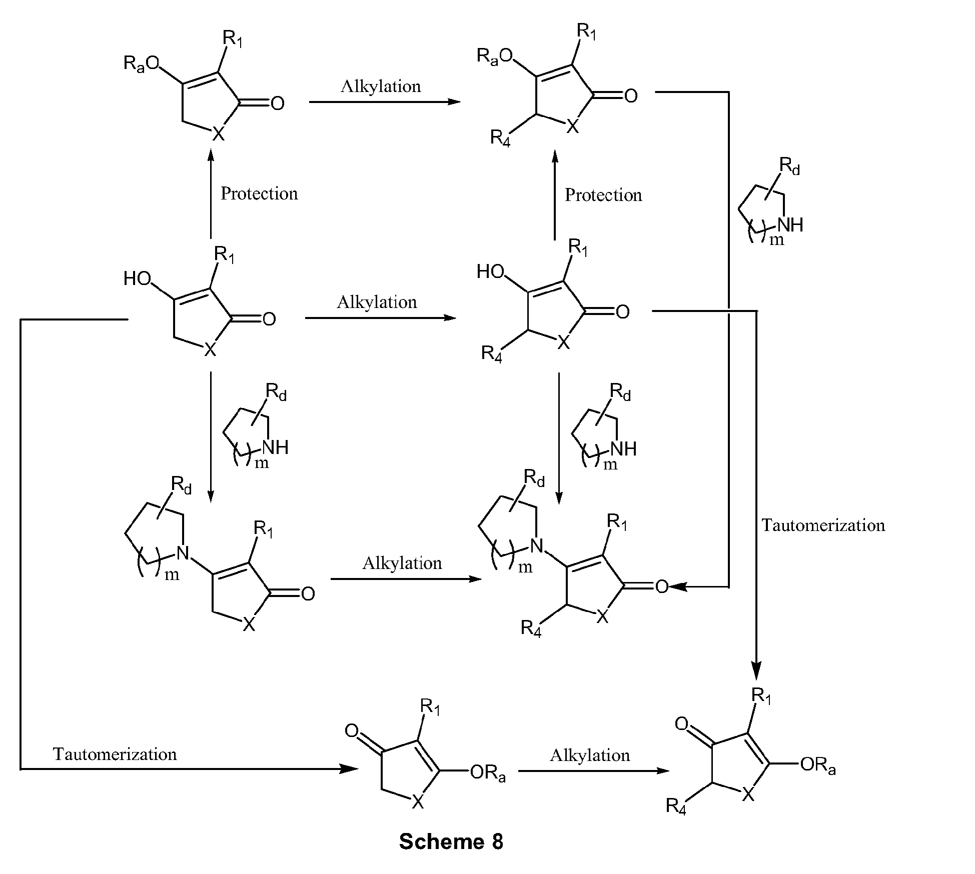

As mentioned above, preferred residues for R₄ are the alkenyl moieties of formula (VII), which may be attached to the compounds of the invention through the alkylation reactions explained above obtaining the compounds of formula (XVI)

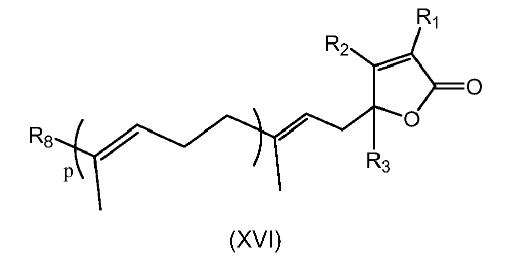

The alkenyl chain of the compounds of formula (XVI) may undergo reactions known for double bonds, such as epoxidation and dihydroxylation which results in further compounds having GSK-3 activity. For example, see scheme 9:

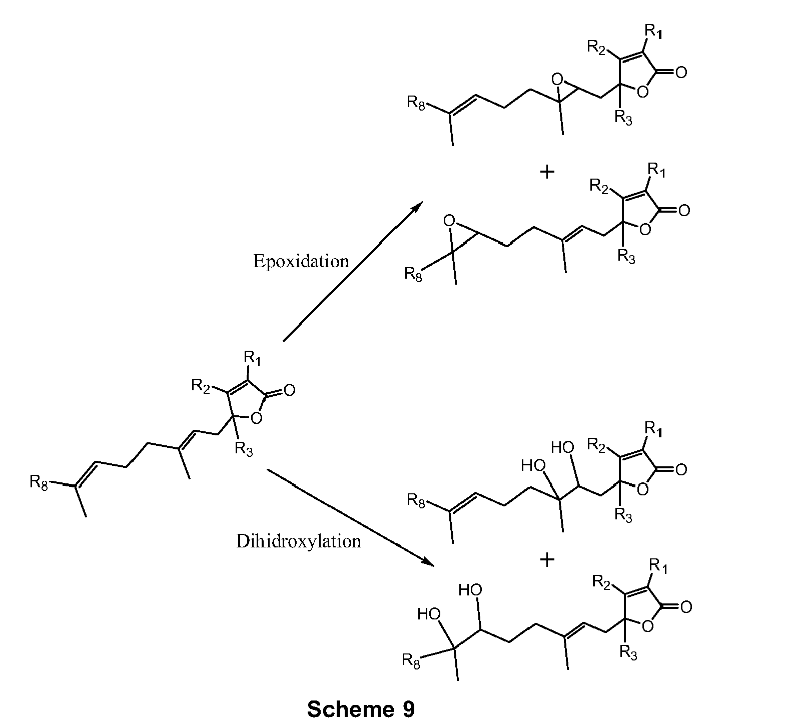

As mentioned above, an embodiment of the invention are compounds of formula (VIII), which are derivatives of ascorbic acid. Said compounds of formula (VIII) are readily available by esterification of ascorbic acid with an ester of formula R₉(C=O)OR_{c}, wherein R₉ is as defined in formula (VIII) and R_{c} is as defined above in formula (IX):

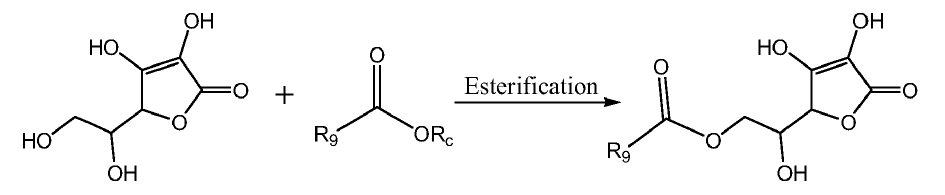

Thus, according to a further aspect, the present invention is directed to a process for the preparation of a compound of formula (I) which comprises in any order one or more of a step selected from the group consisting of:
a) cyclation of a compound of formula (IX) in the presence of a base 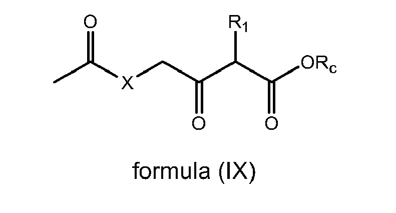 wherein R₁ and X are as defined in formula (I) and OR_{c} forms an ester group, preferably R_{c} being selected from the group consisting of substituted or unsubstituted alkyl, substituted or unsubstituted cycloalkyl, substituted or unsubstituted alkenyl, substituted or unsubstituted cycloalkenyl, substituted or unsubstituted heterocyclyl, substituted or unsubstituted aryl and substituted or unsubstituted arylalkyl;
b) bromination and further heating of a compound of formula (XI) 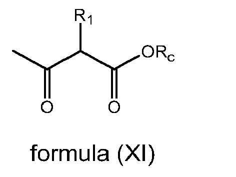 wherein R₁ is as defined in claim 1 and R_{c} is as defined above;
c) alkylation of a compound of formula (XII) 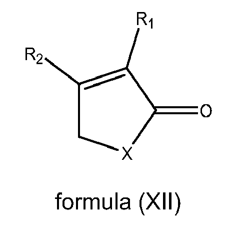 wherein R₁, R₂ and X are as defined in formula (I),
   in the presence of compound of formula R₄-Hal, wherein R₄ is as defined above and Hal is a halogen atom;
d) radical oxidation of a compound of formula (XIII) in the presence of an alcohol of formula Alkyl-OH 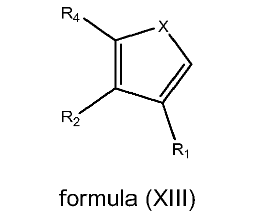 wherein R₁, R₂, R₄ and X are as defined in formula (I); and
e) esterification of ascorbic acid with a compound of formula R₉(C=O)OR_{c}, wherein R₉ is as defined in formula (V) and R_{c} is as defined above.

### Definitions

In the definition of the above mentioned compounds, the following terms have the meaning indicated:
**"Alkyl"** refers to a straight or branched hydrocarbon chain radical consisting of carbon and hydrogen atoms, containing no saturation, having one to twenty carbon atoms, preferably one to twelve, more preferably one to six, and which is attached to the rest of the molecule by a single bond, e. g., methyl, ethyl, n-propyl, i-propyl, n-butyl, t-butyl, n-pentyl, etc.
**"Alkenyl"** refers to a straight or branched hydrocarbon chain radical consisting of carbon and hydrogen atoms, containing at least one unsaturation, having two to twenty carbon atoms, preferably two to twelve carbon atoms, and which is attached to the rest of the molecule by a single bond, e. g., n-propenyl, i-propenyl, n-buten-2-yl, n-buten-3-yl, n-pent-2-yl, n-pent-3-yl, n-pent-4-yl, prenyl, poliprenyl, etc.
**"Alkynyl"** refers to a straight or branched hydrocarbon chain radical consisting of carbon and hydrogen atoms, containing at least one carbon-carbon triple bond, conjugated of not, having two to twenty carbon atoms, preferably two to twelve carbon atoms, and which is attached to the rest of the molecule by a single bond, such as - CCH, -CH₂CCH, -CCCH₃, -CH₂CCCH₃.
**"Cycloalkyl"** refers to a saturated carbocyclic ring having from 3 to 8 carbon atoms, preferably 3 to 6. Suitable cycloalkyl groups include, but are not limited to cycloalkyl groups such as cyclopropyl, cyclobutyl, cyclopentanyl, cyclohexyl, cycloheptyl or cyclooctyl.
**"Cycloalkenyl"** refers to a carbocyclic ring having from 3 to 8 carbon atoms and at least one unsaturation. Suitable cycloalkenyl groups include, but are not limited to cycloalkenyl groups such as 1-cyclobutenyl, 2-cyclobutenyl, 1-cyclopentenyl, 2-cyclopentenyl or 3-cyclopentenyl.
**"Alkoxy"** refers to a radical of the formula -O-alkyl, where alkyl has been previously defined, e. g., methoxy, ethoxy, propoxy, etc.
**"Aryl"** or **"Ar"** refers to a phenyl (also abbreviated as "Ph"), naphthyl, indenyl, fenanthryl or anthracyl radical, preferably phenyl or naphthyl radical.
**"Aralkyl"** refers to an aryl group linked to an alkyl group. Preferred examples include benzyl and phenethyl.
**"Heterocyclyl"** refers to a stable 3- to 15-membered ring which consists of carbon atoms and from one to five heteroatoms selected from the group consisting of nitrogen, oxygen, and sulphur, preferably a 4- to 8-membered ring with one or more heteroatoms, more preferably a 5- or 6-membered ring with one or more heteroatoms. For the purposes of this invention, an heterocyclyl group may be a monocyclic, bicyclic or tricyclic ring system, which may include fused ring systems; and the nitrogen, carbon or sulfur atoms in the heterocyclyl radical may be optionally oxidised; the nitrogen atom may be optionally quaternized; and the heterocyclyl radical may be partially or fully saturated or aromatic. Examples of such heterocycles include, but are not limited to, azepines, benzimidazole, benzothiazole, furan, isothiazole, imidazole, indole, piperidine, piperazine, purine, quinoline, thiadiazole, tetrahydrofuran.

The above mentioned groups (alkyl, alkenyl, alkynyl, aryl, aralkyl an heterocyclyl) may be optionally substituted in at least one position.

References herein to substituted groups in the compounds of the present invention refer to the specified moiety that may be substituted at one or more available positions by one or more suitable groups, e. g., an alkyl as defined above, halogen such as fluoro, chloro, bromo and iodo; cyano; hydroxyl; nitro; azido; -C(=O)NH₂; - C(=O)NHR"; -C(=O)NR"R'''; -C(=O)R"; alkenyl and alkynyl groups including groups having one or more unsaturated linkages and from 2 to about 12 carbon or from 2 to about 6 carbon atoms; -OR"; -OAr, such as phenoxy; -SR"; alkylsulfinyl groups including those moieties having one or more sulfinyl linkages and from 1 to about 12 carbon atoms or from 1 to about 6 carbon atoms; alkylsulfonyl groups including those moieties having one or more sulfonyl linkages and from 1 to about 12 carbon atoms or from 1 to about 6 carbon atoms; -NH₂; -NHR"; -NR"R'" or aryl; wherein R" and R"' are independently selected form alkyl or alkenyl radical as defined above. Unless otherwise indicated, an optionally substituted group may have a substituent at each substitutable position of the group, and each substitution is independent of the other.

Preferred substituents are cyano; alkyl groups, preferably C₁-C₆ alkyl groups; hydroxyl groups whether protected or not; or diols, preferably 1,2-diols whether protected or not.

**"Hydroxyl protecting group"** refers to a group that blocks the -OH function for further reactions and can be removed *in vivo* or under controlled conditions. The hydroxyl protecting groups are well known in the art, representative protecting groups are:
- silyl ethers of formula -Si(R')₃, such as trimethylsilyl ether (also represented as "TMS"), triethylsilyl ether, tert-butyldimethylsilyl ether (also represented as "TBDMSO"), tert-butyldiphenylsilyl ether, tri-isopropylsilyl ether, diethylisopropylsilyl ether, thexyldimethylsilyl ether, triphenylsilyl ether, di-tert-butylmethylsilyl ether;
- alkyl ethers of formula -R', such as methyl ether, tert-butyl ether, benzyl ether, p-methoxybenzyl ether , 3,4-dimethoxybenzyl ether, trityl ether; allyl ether;
- alkoxymethyl ethers of formula -CH₂-O-R', such as methoxymethyl ether, 2-methoxyethoxymethyl ether, benzyloxymethyl ether, p-methoxybenzyloxymethyl ether, 2-(trimethylsilyl)ethoxymethyl ether. The oxygen atom may be replaced by a sulphur atom to form an alkylthiomethyl ether of formula -CH₂-S-R', such as methylthiomethyl ether. Tetrahydropyranyl and related ethers are also commonly used as hydroxyl protecting groups;
- esters of formula -C(=O)R', such as acetate ester, benzoate ester; pivalate ester; methoxyacetate ester; chloroacetate ester; levulinate ester;
- carbonates of formula -C(=O)-O-R', such as benzyl carbonate, p-nitrobenzyl carbonate, tert-butyl carbonate, 2,2,2-trichloroethyl carbonate, 2-(trimethylsilyl)ethyl carbonate, allyl carbonate; or
- sulphates of formula R'S(=O)₂- such as SO_{3.}py or p-tolyl sulphate.

In all the above formula R' represents a group selected from the group consisting of substituted of unsubstituted alkyl, substituted of unsubstituted alkenyl, substituted of unsubstituted alkynyl, substituted of unsubstituted aryl and substituted of unsubstituted aralkyl.

Additional examples of hydroxyl protecting groups can be found in reference books such as Greene and Wuts' "Protective Groups in Organic Synthesis", John Wiley & Sons, Inc., New York, 4th Ed., 2007.

"**Halo**" refers to bromo, chloro, iodo or fluoro.

The compounds of the invention are also meant to include compounds which differ only in the presence of one or more isotopically enriched atoms. For example, compounds having the present structures except for the replacement of a hydrogen by a deuterium or tritium, or the replacement of a carbon by a ¹³C- or ¹⁴C-enriched carbon or ¹⁵N-enriched nitrogen are within the scope of this invention.

The compounds of the present invention represented by the above described formula (I) may include enantiomers depending on the presence of chiral centres or isomers depending on the presence of multiple bonds (e.g. Z, E). The single isomers, enantiomers or diastereoisomers and mixtures thereof fall within the scope of the present invention.

In the following, the present invention is further illustrated by examples. They should in no case be interpreted as a limitation of the scope of the invention as defined in the claims.

### EXAMPLES

### Example 1: synthesis of 5-r3-(tert-Butyl-diphenyl-silanyloxy)-propyll-5-methoxy-5H-furan-2-one

### 1. Synthesis of 3-(tbutyldiphenylsilyloxy)-propanol

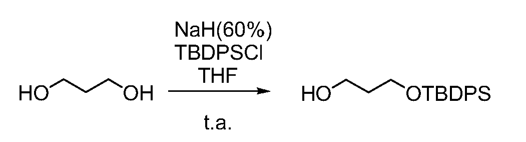

NaH (0.53 g; 13.15 mmol) was added to a solution of the diol (1.00 g; 13.15 mmol) in THF. The mixture was then stirred for 45 minutes at room temperature, and then TBDPSCI (3.61 g; 13.15 mmol) was added, keeping the stirring in the same conditions for 17 additional hours. Et₂O (100 mL) was next added and the organic phase was washed with 10% aq K₂CO₃ solution (3 x 50 mL). The combined organic extracts were dried with Na₂SO₄, filtered and concentrated in vacuum. The obtained crude was finally purified in a chromatography column (mobile phase: 15% AcOEt/Hexano), thus obtaining 1.33 g of the monoprotected diol [74%, yellow oil, Rf: 0.23 (15% AcOEt/Hexane)].
**¹H - RMN** (CDCl₃, δ): 7.56 (4H, m, CHₒ-Ph), 7.25 (6H, m, CH_{p,m}-Ph), 3.69 (2H, t, J = 5.8 Hz, CH₂-3), 3.65 (2H, t, J = 5.8 Hz, CH₂-1), 2.73 (1H, s, OH), 1.68 (2H, q, J = 5.8 Hz, CH₂-2), 0.94 (9H, s, CH₃-^{t}Bu).
**¹³C-RMN** (CDCl₃, δ): 136.90 (CHₒ-Ph), 133.63 (C-Ph), 130.18 (CHₚ-Ph), 128.16 (CHₘ-Ph), 63.72 (CH₂-1), 62.43 (CH₂-3), 34,63 (CH₂-3), 27.22 (CH₃-^{t}Bu), 19.48 (C-^{t}Bu).

### 2. Synthesis of 1-(^{t}butyldiphenylsilyloxy)-3-iodinetane

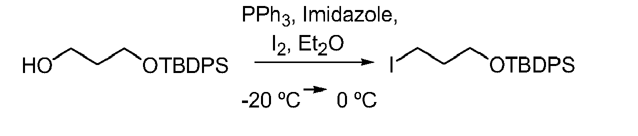

PPh₃ (1.36 g; 5.16 mmol) and imidazole (0.90 g; 13.15 mmol) were added to a solution of the monoprotected diol (1.26 g; 4.00 mmol) in THF (20 mL), and the mixture was stirred until complete dissolution. Subsequently, the solution was cooled to -20°C and then I₂ (1.21 g; 4.75 mmol) was added, appearing an orange coloration in the solution. The stirring was carried on, in the same conditions, for 15 minutes. Then, the cool bath was removed so that the mixture could reach the room temperature. After 1 hour and 30 minutes, the flask was immersed into a water-ice bath and the reaction was stopped by adding a saturated solution of NaHCO₃ (10 mL). A white pellet was formed, while the solution took a transparent yellow colour. The solid was next separated by filtration, the pellet was washed with Et₂O (50 mL) and the aqueous phase was extracted with Et₂O (2 x 50 mL). The mixed organic phases were washed with 10% Na₂S₂O₄ solution (20 mL) and H₂O (20 mL), dried with Na₂SO₄, filtered and concentrated in vacuum. The crude thus obtained was finally purified in a chromatography column (mobile phase: 100 % CH₂Cl₂), obtaining 1.12 g of the iodide [66 %, colourless oil, Rf: 0.83 (100 % CH₂Cl₂)].
**¹H - RMN** (CDCl₃, δ): 8.04 (4H, m, CHₒ-Ph), 7.67 (6H, m, CH_{p,m}-Ph), 4.03 (2H, t, J = 5.7Hz, CH₂-3), 3.60 (2H, t, J = 6.8Hz, CH₂-1), 2.38 (2H, q, J = 5.9Hz, J = 6.4Hz, CH₂-2), 1.43 (9H, s, CH₃-^{t}Bu).
**¹³C - RMN** (CDCl₃, δ): 133.4 (CHₒ-Ph), 133.08 (C-Ph), 129.39 (CHₚ-Ph), 127.42 (CHₘ-Ph), 62.81 (CH₂-3), 35.71 (CH₂-2), 26.63 (CH₃-^{t}Bu), 18.88 (C-^{t}Bu), 3.06 (CH₂-1).

### 3. Synthesis of 2-[(3-^{t}butyldiphenilsilyloxy)propyl]furan

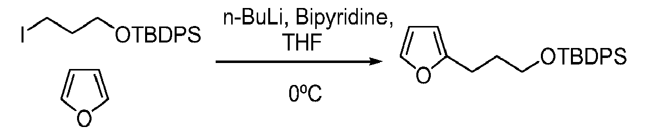

N-BuLi 2.5 M in hexane (0.068 mL; 0.94 mmol) was slowly added to a solution of furan (0.064 g; 0.94 mmol) and 2,2'-bipyridine (catalytic amount) in THF (2 mL), cooled at 0 °C. The mixture was next stirred for 1 hour under these conditions. Then,, iodine (0.20 g; 0.47 mmol) solved in THF (1 mL) was added, and the stirring maintained at room temperature for 4 hours. The reaction was then stopped by adding a saturated NH₄Cl solution (2 mL) to the mixture in a bath at 0°C. An extraction with hexane was next performed (2 x 3ml) and the mixed organic extracts dried with Na₂SO₄, filtered and concentrated in vacuum. The crude thus obtained was finally purified in a chromatography column (mobile phase: hexane), obtaining 0.147 g of the alkylfuran [86 %, yellow oil, Rf: 0.73 (10 % AcOEt/Hexane)].
**¹H - RMN (** CDCl₃, δ) : 7.69 (4H, dd, J = 5.8 Hz, J = 1.6 Hz, CHₒ-Ph), 7.42 (6H, m, CH_{p,m}-Ph), 7.30 (1 H, s , CH-5 furanyl), 6.28 (1 H, dd, J = 2.9 Hz, J = 1.7 Hz, CH-4 furanyl), 5.96 (1 H, dd, J = 2.6 Hz, J = 2.0 Hz, CH-3 furanyl), 3.72 (2H, m, CH₂-3), 2.78 (2H, t, J = 7.7 Hz, CH₂-1), 1.91 (2H, m, CH₂-2), 1.08 (9H, s, CH₃-tBu).
**¹³C - RMN** ( CDCl₃, δ): 155.91 (CH-2 furanyl), 140.72 (CH-5 furanyl), 135.54 (CHₒ-Ph), 133.84 (C-Ph), 129.55 (CHₚ-Ph), 127.61 (CHₘ-Ph), 110.43 (CH-4 furanyl), 104.77 (CH-3 furanyl), 62.89 (CH₂-3), 30.81 (CH₂-2), 26.82 (CH₃-^{t}Bu), 24.35 (CH₂-1), 19.22 (C-^{t}Bu).

### 4. Synthesis of 5-[(3(^{t}butyldiphenylsilyloxy)propyl)]- 5-methoxy-5H- furan-2-one

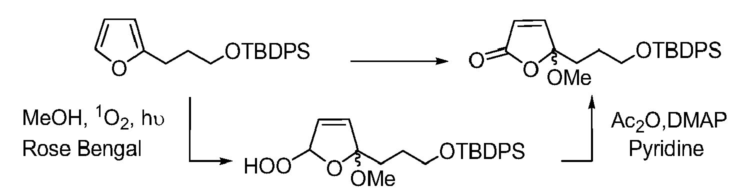

A solution of alquilfuran (12.89 g; 35.38 mmol) and rose Bengal (0.89 g) in MeOH (200mL) was cooled at -79°C and radiated with a 200 W UV lamp. The mixture was next stirred in O₂ atmosphere for 4 hours and, afterwards, the mixture was allowed to reach the room temperature. The solvent was evaporated in vacuum. The obtained crude was finally purified in a chromatography column (mobile phase: 60% AcOEt/Hexane), to retain the catalyser rose Bengal, thus obtaining 16.24 g of impure hydroperoxide [light yellow oil, Rf: 0.7 (30% AcOEt/Hexane)].

Ac₂O (22.81 mL) was added dropwise to a pink solution of the non-purified hydroperoxide (16.24 g) and DMAP (catalytic amount) in pyridine (91.29 mL). The reaction mass changed to a dark orange colour. When the reaction had finished, the mixture was stirred for 23 hours at room temperature, and then MeOH (100 mL) was added. The stirring was carried on for 20 minutes. Once the MeOH was evaporated in vacuo, Et₂O (2 x 75mL) was added and the mixture was washed with 10% CuSO₄ solution (3 x 100 mL). The mixed organic extracts were dried with Na₂SO₄ and concentrated in vacuum. The obtained crude was finally purified in a chromatography column (mobile phase: 5% AcOEt/Hexane), obtaining 12.64 g of the butenolide [81%, Rf: 0.46 (20% AcOEt/Hexane)].
**¹H - RMN** (CDCl₃, δ) : 7.81 (4H, m, CHₒ-Ph), 7.56 (6H, m, CH_{p,m}-Ph), 7.24 (1 H, dd, J = 5.7 Hz, CH-4 furanone), 6.36 (1H, dd, J= 5.7 Hz, CH-3 furanone), 3.83 (2H, t, J = 6.1 Hz, CH₂-3), 3.37 (3H, s, OCH₃), 2.18 (2H, m, CH₂-1), 1.80 (2H, m, CH₂-2).
**¹³C - RMN** (CDCl₃, δ):170.32 (CO), 153.89 (CH-4 furanone), 135.94 (CHₒ-Ph), 134.13 (C-Ph), 130.06 (CHₚ-Ph), 128.03 (CHₘ-Ph), 125.22 (CH-3 furanone), 111.58 (C-5 furanone), 63.64 (CH₂-3), 51.54 (OCH₃), 33.95 (CH₂-1), 27.27 (CH₃-tBu), 26.82 (CH₂-2), 19.61 (C - tBu).

### Example 2: Synthesis of 5-[4-(tert-Butyl-diphenyl-silanyloxy)-butyl]-5-methoxy-5H-furan-2-one

### 1. Synthesis of 4-(^{t}butyldiphenylsilyloxy)butanol

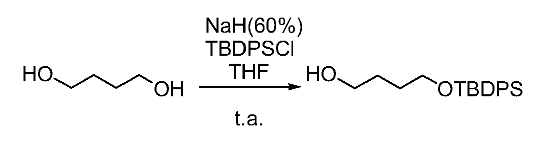

NaH (0.88 g; 22.19 mmol) was added to a solution of diol (2.00 g; 22.19 mmol) in THF (40 mL). The mixture was next stirred for 45 minutes at room temperature, and then TBDPSCI (6.10 g; 22.19 mmol) was added. The stirring was kept in the same conditions for 16 additional hours. Next, Et₂O (100 mL) was added to the mixture and the organic phase was washed with 10% K₂CO₃ solution (3 x 50 mL). The mixed organic extracts were dried with Na₂SO₄, filtered and concentrated in vacuum. The obtained crude was finally purified in a chromatography column (mobile phase: 12% AcOEt/Hexane), obtaining 4.56 g of the monoprotected diol [63%, white solid, Rf: 0.20 (15% AcOEt/Hexane)].
**¹H - RMN** (CDCl₃, δ): 7.67 (4H, m, CHₒ-Ph), 7.30 (6H, m, CH_{p,m}-Ph), 3.67 (2H, t, J= 5.6Hz, CH₂-1), 3.56 (2H, t, J= 5.8Hz, CH₂-4), 3.40 (1H, s, OH), 1.62 (4H, m, CH₂-2, CH₂-3), 1.06 (9H, s, CH₃-^{t}Bu).
**¹³C - RMN** (CDCl₃, δ): 136.00 (CHₒ-Ph), 134.11 (C-Ph), 130.08 (CHₚ-Ph), 128.10 (CHₘ-Ph), 64.45 (CH₂-1), 63.16 (CH₂-4), 30.19 (CH₂-2), 29.69 (CH₂-3), 27.28 (CH₃-^{t}Bu), 19.60 (C-^{t}Bu).

### 2. Synthesis of 1-(^{t}butyldiphenylsilyloxy)-4-iodinebutane

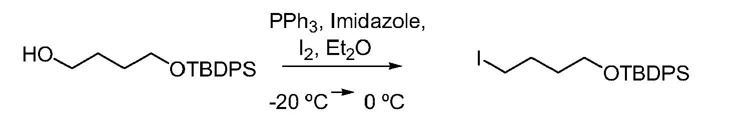

PPh₃ (1.94 g; 7.40 mmol) and imidazole (1.26 g; 18.54 mmol) were added to a solution of the monoprotected diol (2.06 g; 6.26 mmol) in THF (26 mL), while stirring the mixture until complete dissolution. Subsequently, the mixture was cooled to -20°C and, afterwards, I₂ (1.77 g; 6.96 mmol) was added. An orange colouration was formed.. The stirring was carried on for 15 additional minutes, and then the cool bath was removed to allow the mixture to reach the room temperature for 30 minutes. After conventionally processing the reaction mixture, the obtained crude was purified in a chromatography column (mobile phase: 100 % CH₂Cl₂), thus obtaining 2.30 g of the iodine [86 %, yellow oil, Rf: 0.81 (100 % CH₂Cl₂)].
**¹H - RMN** (CDCl₃, δ): 7.96 (4H, m, CHₒ-Ph), 7.68 (6H, m, CH_{p,m}-Ph), 3.98 (2H, t, J= 6.1 Hz, CH₂-4), 3.47 (2H, t, J= 6.5Hz, CH₂-1), 2.24 (2H, t, J= 6.0Hz, CH₂-2), 1.94 (2H, m, CH₂-3), 1.35 (9H, s, CH₃-^{t}Bu).
**¹³C**-**RMN**(CDCl₃,δ): 135.47 (CHₒ-Ph), 133.71 (C-Ph), 129.55 (CHₚ-Ph), 127.60 (CHₘ-Ph), 62.60 (CH₂-4), 33.20 (CH₂-2), 30.08 (CH₂-3), 26.82 (CH₃-^{t}Bu), 19.14 (C-^{t}Bu), 6.96 (CH₂-1).

### 3. Synthesis of 2-[(4-^{t}butyldiphenylsilyloxy)butyl]furan

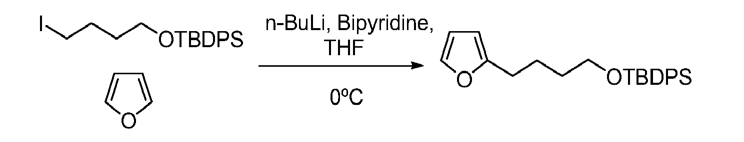

n-BuLi 2.5 M in hexane (4.20 mL; 10.48 mmol) was slowly added to a solution of furan (0.71 g; 10.48 mmol) and 2,2'-bipyridine (catalytic amount) in THF (20 mL), cooled at 0 °C. The mixture was then stirred for 1 hour in these conditions. Afterwards, the iodine (2.30 g; 5.24 mmol) solved in THF (3 mL) was added to the mixture and the stirring was maintained at room temperature for 5 hours. Once the reaction was processed as usual, the obtained crude was finally purified in a chromatography column (mobile phase: Hexane), thus obtaining 1.49 g of the alkyl furan [71%, yellow oil, Rf: 0.72 (10 % AcOEt/Hexane)].
**¹H - RMN** ( CDCl₃, δ) : 8.11 (4H, m, CHₒ-Ph), 7.77 (6H, m, CH_{p,m}-Ph), 7.64 (1 H, s, CH-5 furanyl), 6.63 (1 H, s , CH-4 furanyl), 6.34 (1 H, s , CH-3 furanyl), 4.12 (2H, t, J = 6.4 Hz, CH₂-1), 3.01 (2H, t, J = 7.5 Hz, CH₂-4), 2.20 (2H, m, CH₂-3), 2.03 (2H, m, CH₂-2), 1.50 (9H, s, CH₃-tBu).
**¹³C - RMN** ( CDCl₃, δ): 155.98 (CH-2 furanyl), 140.50 (CH-5 furanyl), 135.50 (CHₒ-Ph), 133.91 (C-Ph), 130.16 (CHₚ-Ph), 127.74 (CHₘ-Ph), 109.97 (CH-4 furanyl), 104.72 (CH-3 furanyl), 63.46 (CH₂-4), 31.93 (CH₂-2), 27.58 (CH₃-^{t}Bu), 26.55 (CH₂-1), 24.30 (CH₂-2), 19.15 (C-^{t}Bu).

### 4. Synthesis of 5-[(4(^{t}butyldiphenylsilyloxy)butyl)]- 5-methoxy-5H- furan-2-one

A solution of alkyl furan (1.59 g; 4.18 mmol) and rose Bengal (0.099 g) in MeOH 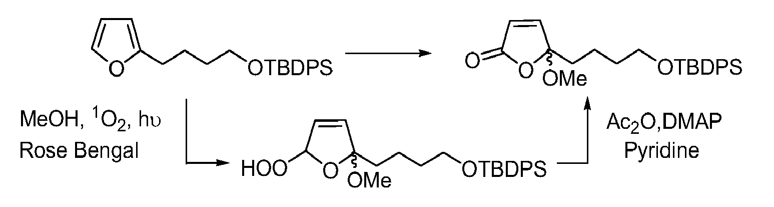 (62 mL) was cooled at -79 °C, and irradiated with a 200 W UV lamp. The mixture was next stirred in O₂ atmosphere for 4 hours and 30 minutes. Afterwards, the mixture was allowed to reach room temperature and next the solvent was evaporated in vacuum. The obtained crude was finally purified in a chromatography column, to retain the catalyser rose Bengal, thus obtaining 1.95 g of the impure hydroperoxide [light yellow oil, Rf: 0.4 (20% AcOEt/Hexane)].

Ac₂O (2.88 mL) was added dropwise to a pink solution of the non-purified hydroperoxide (1.95 g) and DMAP (catalytic amount) in pyridine (8.55 mL), observing that the reaction mass changes to a dark orange colour. After the addition, the mixture was stirred for 14 hours at room temperature; next, MeOH (19 mL) was added. The stirring was carried on for 20 minutes. Once the reaction was finished, 1.59 g of the butenolide was obtained [90%, brown oil, Rf: 0.32 (20% AcOEt/Hexane)].
**¹H - RMN** (CDCl₃, δ) : 7.63 (4H, m, CHₒ-Ph), 7.40 (6H, m, CH_{p,m}-Ph), 7.08 (1 H, dd, J= 1.0Hz, J= 5.7Hz, CH-4 furanona), 6.19 (1H, dd, J= 1.0Hz, J= 5.6Hz, CH-3 furanona), 3.63 (2H, t, J= 6.2Hz, CH₂-1), 3.20 (3H, s, OCH₃), 1.88 (2H, t, J= 7.5Hz, CH₂-4), 1.57 (1 H, m, CH₂-2), 1.47 (1 H, m, CH₂-3), 1.02 (9H, s, CH₃-^{t}Bu).
**¹³C - RMN** (CDCl₃, δ):170.00 (CO), 153.46 (CH-4 furanona), 135.53 (CHₒ-Ph), 133.84 (C-Ph), 129.57 (CHₚ-Ph), 127.61 (CHₘ-Ph), 124.81 (CH-3 furanona), 111.21 (C-5 furanona), 63.34 (CH₂-1), 51.14 (OCH₃), 36.62 (CH₂-4), 32.21 (CH₂-2), 26.82 (CH₃-tBu), 19.71 (CH₂-3), 19.18 (C - tBu).

### Example 3: Synthesis of 5-[6-(tert-Butyl-diphenyl-silanyloxy)-hexyl]-5-methoxy-5H-furan-2-one

### 1. Synthesis of 6-(^{t}butyldiphenylsilyloxy)hexanol

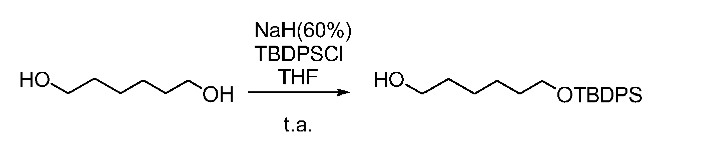

NaH (1.24 g; 33.84 mmol) was added to a solution of the diol (4.00 g; 33.84 mmol) in THF (56 mL). The mixture was stirred for 45 minutes at room temperature. Then, TBDPSCI (9.30 g; 33.84 mmol) was added, keeping the stirring in the same conditions for 19 additional hours, then the reaction was processed as usual. The obtained crude was finally purified in a chromatography column (mobile phase: 5% AcOEt/Hexane), thus obtaining 2.58 g of the monoprotected diol [21%, colourless oil, Rf: 0.30 (25% AcOEt/Hexane)].
**¹H - RMN** (CDCl₃, δ): 7.95 (4H, m, CHₒ-Ph), 7.66 (6H, m, CH_{p,m}-Ph), 3.94 (2H, t, J= 6.5Hz, CH₂-1), 3.87 (2H, t, J= 6.5Hz, CH₂-6), 1.84 (5H, m, CH₂-2, CH₂-5, OH), 1.64 (4H, m, CH₂-3, CH₂-4), 1.33 (9H, s, CH₃-^{t}Bu).
**¹³ C-RMN** (CDCl₃, δ): 135.52 (CHₒ-Ph), 134.07 (C-Ph), 129.53 (CHₚ-Ph), 127.53 (CHₘ-Ph), 63.80 (CH₂-1), 62.83 (CH₂-6), 32.67 (CH₂-2), 32.45 (CH₂-5), 26.83 (CH₃-^{t}Bu), 25.54 (CH₂-3), 25.42 (CH₂-4), 19.17 (C-^{t}Bu).

### 2. Synthesis of 1-tbutyldiphenylsiyiloxy)-6-iodinehexane

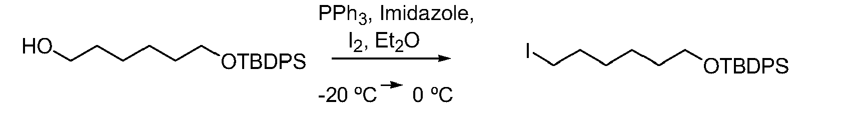

PPh₃ (1.76 g; 6.48 mmol) and imidazole (1.14 g; 16.70 mmol) were added to a solution of the protected diol (1.90 g; 5.33 mmol) in THF (20 mL). The mixture was stirred until complete dissolution. Subsequently, the mixture was cooled to -20 °C and then I₂ (1.56 g; 6.14 mmol) was added, observing an orange colouration. The stirring was carried on for 15 minutes in the same conditions. Next, the reaction mixture was removed from the cool bath so that it could reach the room temperature. After 30 minutes, the reaction was finished. The obtained crude was purified in a chromatography column (mobile phase: 10% CH₂Cl₂), thus obtaining 2.30 g of the iodide [93%, yellow oil, Rf: 0.89 (100 % CH₂Cl₂)].
**¹H - RMN** (CDCl₃, δ): 7.95 (4H, m, CHₒ-Ph), 7.61 (6H, m, CH_{p,m}-Ph), 3.81 (2H, t, J= 6.4Hz, CH₂-6), 3.23 (2H, t, J= 7Hz, CH₂-1), 1.89 (2H, t, J= 7Hz, CH₂-2), 1.69 (2H, t, J= 6.5Hz, CH₂-5),1.49 (4H, m, CH₂-3, CH₂-4), 1.30 (9H, s, CH₃-^{t}Bu).
**¹³C - RMN** (CDCl₃, δ): 135.33 (CHₒ-Ph), 133.78 (C-Ph), 129.35 (CHₚ-Ph), 127.43 (CHₘ-Ph), 63.46 (CH₂-6), 33.27 (CH₂-2), 32.08 (CH₂-5), 29.98 (CH₂-3) 26.76 (CH₃-^{t}Bu), 24.53 (CH₂-4), 19.02 (C-^{t}Bu), 6.81 (CH₂-1).

### 3. Synthesis of 2-[(6-^{t}Butyldiphenylsilyloxy)hexyl]furan

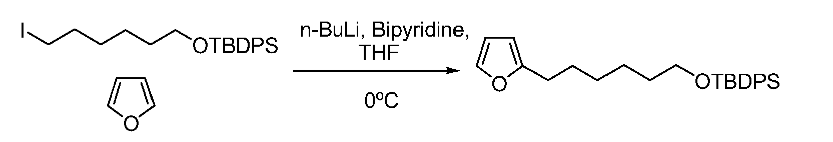

n-BuLi 2.5 M in hexane (3.75 mL; 9.38 mmol) was slowly added to a solution of furan (0.64 g; 9.38 mmol) and 2,2'-bipyridin (catalytic amount) in THF (38 mL), cooled at 0 °C. The mixture was stirred for 1 hour in these conditions. Afterwards, the iodide (2.19 g; 4.69 mmol) solved in THF (5 mL) was added to the previous mixture. The stirring at room temperature was carried on for 5 hours, and afterwards the reaction mixture was conventionally processed. The obtained crude was purified in a chromatography column (mobile phase: Hexane → 1 % AcOEt/Hexane → 3% AcOEt/Hexane), thus obtaining 1.35 g of the alkyl furan [71 %, yellow oil, Rf: 0.7 (10% AcOEt/Hexane)].
**¹H - RMN** ( CDCl₃, δ) : 7.72 (4H, m, CHₒ-Ph), 7.43 (6H, m, CH_{p,m}-Ph), 7.33 (1 H, d, J= 1 Hz , CH-5 furanyl), 6.30 (1 H, t , J= 2.45Hz, CH-4 furanyl), 6.00 (1 H, d, J= 3.0Hz, CH-3 furanyl), 3.67 (2H, t, J = 6.4 Hz, CH₂-1), 2.63 (2H, t, J= 7.6Hz, CH₂-6), 1.60 (4H, m, CH₂-5, CH₂-2), 1.38 (4H, m, CH₂-4, CH₂-3), 1.09 (9H, s, CH₃-tBu).
**¹³C - RMN** ( CDCl₃, δ): 156.49 (CH-2 furanyl), 140.60 (CH-5 furanyl), 135.54 (CHₒ-Ph), 134.06 (C-Ph), 129.47 (CHₚ-Ph), 127.55 (CHₘ-Ph), 110.00 (CH-4 furanyl), 104.51 (CH-3 furanyl), 63.83 (CH₂-1), 32.41 (CH₂-2), 28.87 (CH₂-6), 27.97 (CH₂-5), 27.88 (CH₂-4), 26.83 (CH₃-^{t}Bu), 25.48 (CH₂-3), 19.19 (C-^{t}Bu).

### 4. Synthesis of 5-[(6(^{t}butyldiphenylsilyloxy)hexyl)]- 5-methoxy-5H- furan-2-one

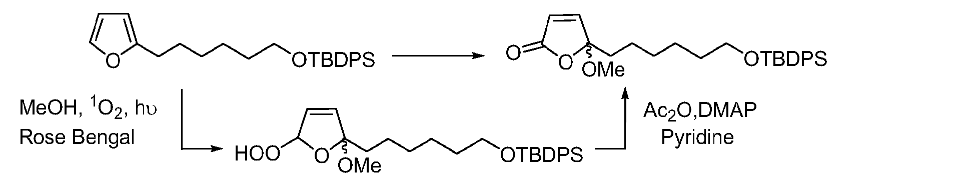

A solution of alkyl furan (1.30 g; 3.19 mmol) and rose Bengal (0.075 g) in MeOH (47 mL) was cooled at -79 °C, and was irradiated with a 200 W UV lamp. The mixture was stirred in O₂ atmosphere for 4 hours and 30 minutes. Afterwards, the mixture was allowed to reach the room temperature. The solvent was next evaporated in vacuum. The obtained crude was finally purified in a chromatography column, to retain the catalyser rose Bengal, thus obtaining 1.66 g of the impure hydroperoxide [light yellow oil, Rf: 0.74 (10% AcOEt/Hexane)].

Ac₂O (2.20 mL) was added dropwise to a pink solution of the non-purified hydroperoxide (1.66 g) and DMAP (catalytic amount) in pyridine (6.52 mL), observing that the reaction mass changes to a dark orange colour. Finished the addition, the mixture was stirred for 14 hours at room temperature; afterwards, MeOH (20 mL) was added and the stirring was carried on for 20 minutes. Once the reaction was processed, 1.24 g of the butenolide was obtained [86%, brown oil, Rf: 0.43 (25% AcOEt/Hexane)].

### Example 4: Synthesis of 5-(6-Furan-3-yl-3-methyl-hex-2-enyl)-4-methoxy-3-methyl-5H-furan-2-one

(See previous examples for the synthesis of compounds **1** and **2**)

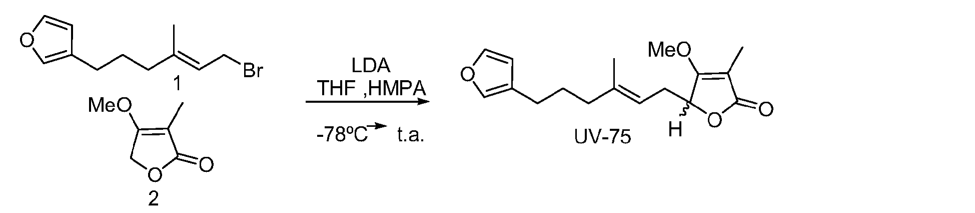

N-BuLi (2,5 M) (187 µl; 468 µmol) and HMPA (270 µl ;1.55 mmol) were added dropwise to a diisopropilamine solution (69 µl; 492 µmol) in THF (0.5 ml), immersed in an acetone/CO₂ bath. The mixture was stirred for 25 minutes. The solution changed to a yellowish colour. Next, the butenolide **2** (48.2 mg; 375 µmol) solved in THF (0.5 ml) was added to the mixture and said mixture was stirred for 30 additional minutes. Afterwards, the alylic bromide **1** (34.1 mg; 187 µmol) and HMPA (67 µl; 385 µmol) solved in THF (1 ml) were added to the mixture, and said mixture was stirred all night while it slowly reached room temperature. The reaction was stopped by the addition of saturated NH₄Cl. Next, an extraction with a mixture of 10% MeOH/CH₂Cl₂ (10 x 10 ml) was performed. The mixed organic extracts were dried with Na₂SO₄, filtered and concentrated in vacuum. The obtained crude was finally purified in a chromatography column (mobile phase: 5% AcOEt/Hexane), obtaining 15.5 mg [35 %, transparent liquid, Rf: 0.20 (30% AcOEt/Hexane)]
**¹H - RMN** (CDCl₃, δ) : 7.33 (1H, s, CH-5 furanyl), **7.20 (1H, s, CH-2 furanyl), 7.19 (1H, s, CH-2 furanyl), 6.26 (1 H, s, CH-4 furanyl), 6.24 (1 H, s, CH-4 furanyl),** 5.08 (1 H, t, J= 8.20 Hz, CH-2'), **4.61 (1H, s, CH-5), 4.56 (1H, s, CH-5), 4.07 (3H, s, -OCH₃), 4.06 (3H, s, -OCH₃),** 2.59 (1H, m, CH-1'), 2.35 (3H, m,CH-1',CH₂-6'), 2.01 (2H, m, CH₂-4), **1.97 (3H, s, CH₃-3), 1.95 (3H, s, CH₃-3),** 1.65 (2H, m, CH₂-5'), **1.64 (3H, s, CH₃-3'), 1.61 (3H, s, CH₃-3').**
**¹³C - RMN** (CDCl₃, δ): 174.86 (C-4), 172.98 (C=O), 142.64 (CH-5 furanyl), 139.38 (C-3'), 138.81 (CH-2 furanyl), 125.05 (C-3 furanyl), 116.72 (CH-2'), 110.96 (CH-4 furanyl), 97.71 (C-3), 77.19 (CH-5), 58.68 (-OCH₃), **39.15** and **38.98 (CH₂-4'),** 29.69 (CH₂-5'), 28.03 (CH₂-1'), **24.57 y 24.07 (CH₂-6'),** 16.10 (CH₃-3'), 8.37 (CH₃-3).

The signals corresponding to the isomers are shown in **bold.**

### Example 5: Synthesis of Dodecanoic acid 2-(3,4-dihydroxy-5-oxo-2,5-dihydro-furan-2-yl)-2-hydroxy-ethyl ester

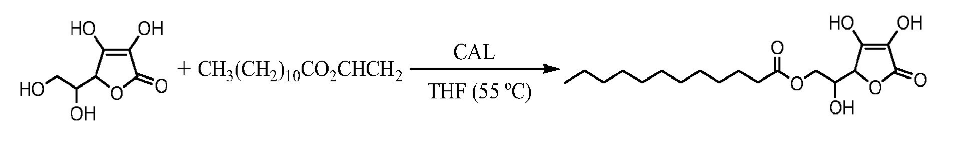

*Candida Antarctica Lipase* (CAL) (50 mg) was added to a solution of 10-Undecenoic acid vinyl ester (0.210 g, 1 mmol) and ascorbic acid (0.176 g, 1 mmol) in THF anhydrous (3 mL) in a capped vial. The mixture was incubated (55 °C, 250 rpm) for 24 hours.

The enzyme was removed by filtration and the mixture was washed with water (15 mL) and extracted with ethyl acetate (15 mL). The organic layer was dried over anhydrous sodium sulfate, filtered and concentrated under reduced pressure to give (0.176 g, 50%) of the aimed compound as a white solid.
**¹H RMN** (400 MHz, MeOD): 4.73 (d, 1 H, J= 2.0Hz, CH), 4.22 (ddd, 2H, J=6.4Hz, J=11.1Hz, J=16.9Hz, CH₂), 4.08 (ddd, 1H, J=2.1Hz, J=5.8Hz, J=7.1Hz, CH), 2.37 (t, 2H, J=7.4Hz, CH₂), 1.67-1.55 (m, 2H, CH₂), 1.29 (s, 16H, 8CH₂), 0.89 (t, 3H, J=6.9Hz, CH₃)
**¹³C RMN** (100 MHz, MeOD): 173.9 (CO), 171.9 (CO), 152.8 (C-OH), 118.9 (C-OH), 76.0 (CH), 66.9 (CH₂O), 64.4 (CH-OH), 33.7 (CH₂), 31.9 (CH₂), 29.5 (CH₂), 29.4 (CH₂), 29.3 (CH₂), 29.2 (CH₂), 29.0 (CH₂), 24.8 (CH₂), 22.5 (CH₂), 13.5 (CH₃).

### Example 6: Synthesis of Tetradecanoic acid 2-(3,4-dihydroxy-5-oxo-2,5-dihydro-furan-2-yl)-2-hydroxy-ethyl ester

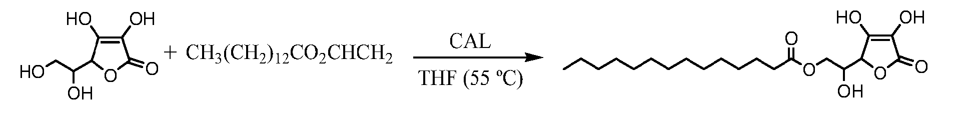

*Candida Antarctica Lipase* (CAL) (50 mg) was added to a solution of Vinyl miristate (0.254 g, 1 mmol) and ascorbic acid (0.176 g, 1 mmol) in THF anhydrous (3 mL) in a capped vial. The mixture was incubated (55 °C, 250 rpm) for 24 hours.

The enzyme was removed by filtration and the mixture was washed with water (15 mL) and extracted with ethyl acetate (15 mL). The organic layer was dried over anhydrous sodium sulfate, filtered and concentrated under reduced pressure to give (0.270 g, 72%) of the aimed compound as a white solid.
**¹H-RMN** (400 MHz, MeOD): 4.65 (s, 1 H, CH-O), 4.22 (ddd, 2H, J=6.5Hz, J=11.1Hz, J=16.8Hz, CH₂), 4.08 (m, 1 H, CH), 2.36 (t, 2H, J=7.4Hz, CH₂), 1.62 (m, 2H, CH₂), 1.30 (m, 20H, 10CH₂), 0.89 (t, 3H, J=6.5Hz, CH₃).
**¹³C-RMN** (100 MHz, MeOD): 174.0 (CO), 172.1 (CO), 152.7 (C-OH), 119.5 (C-OH), 76.1 (CH), 66.5 (CH₂), 64.2 (CH), 33.8 (CH₂), 32.0 (CH₂), 29.3 (CH₂), 29.2 (CH₂), 29.1 (CH₂), 29.0 (CH₂), 28.9 (CH₂), 28.8 (CH₂), 29.7 (CH₂), 29.6 (CH₂), 25.2 (CH₂), 22.1 (CH₂), 13.2(CH₃).

### Example 7: Synthesis of 2-Butenoic acid 2-(3,4-dihydroxy-5-oxo-2,5-dihydro-furan-2-yl)-2-hydroxy-ethyl ester

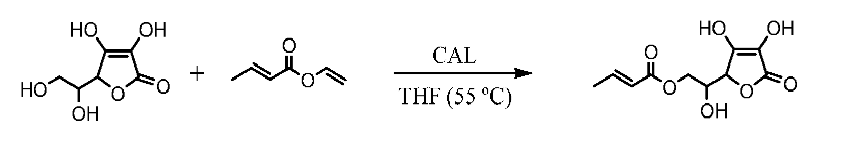

*Candida Antarctica Lipase* (CAL) (50 mg) was added to a solution of Vinyl crotonate (0.11 g, 1 mmol) and ascorbic acid (0.176 g, 1 mmol) in THF anhydrous (3 mL) in a capped vial. The mixture was incubated (55 °C, 250 rpm) for 24 hours.

The enzyme was removed by filtration and the mixture was washed with water (15 mL) and extracted with ethyl acetate (15 mL). The organic layer was dried over anhydrous sodium sulfate, filtered and concentrated under reduced pressure to give (0.185 g, 76%) of the aimed compound as a white solid.
**¹H-RMN** (400 MHz, MeOD): 7.05 (qd, 1 H, J=6.9Hz, J=14.0Hz, CH), 5.91 (d, 1 H, J=15.6Hz, CH) 4.76 (s, 1 H, CH), 4.26 (m, 2H, CH₂), 4.11 (m, 1 H, CH), 1.89 (d, 3H, J=6.9 Hz, CH₃).
**¹³C-RMN** (100 MHz, MeOD): 173.1 (CO), 172.0 (CO), 167.2 (CH), 153.1 (C-OH), 146.0 (CH), 122.0 (C-OH), 76.2 (CH), 67.1 (CH₂), 64.3 (CH), 17.1 (CH₃).

### Example 8: Synthesis of [4-(3-Hydroxy-4-methyl-5-oxo-2,5-dihydro-furan-2-ylmethyl)-phenyl]-acetic acid 2-oxo-2-phenyl-ethyl ester

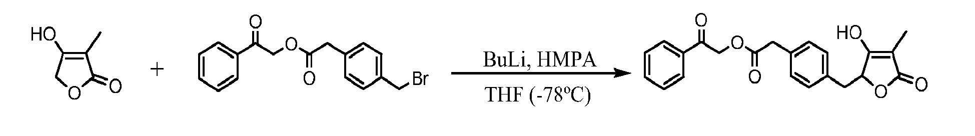

N-butyllithium 1.6M in hexane (0.53 mL; 5.79 mmol) was added dropwise under nitrogen atmosphere to a solution of 4-hydroxy-3-methyl-5H-furan-2-one (0.3 g; 2.63 mmol) and hexamethylphosphoramide in anhydrous THF (75 mL) which had been previously cooled at -78 °C.. The resulting mixture was stirred at -78 °C for 2 h. Then, (4-Bromomethyl-phenyl)-acetic acid 2-oxo-2-phenyl-ethyl ester (0.95 g; 2.76 mmol) was added to the solution at -78 °C. Stirring was kept under these conditions for 1 hour, and then the solution was allowed to warm to room temperature and the stirring was maintained for 15 h. The reaction mixture was next diluted with ethyl acetate (200 mL). The solution was then acidized with HCl 1 N until the pH became 5. The organic layer was washed with water (200 mL) and brine (200 mL), dried with sodium sulphate, filtered and concentrated in vacuum. The residue was finally purified by column chromatography using *n*-hexane/AcOEt (1:1) to give 0,050 g (5%) of the aimed compound as yellow oil.
**¹H RMN** (400 MHz, CDCl₃): 7.82 (d, 2H, J= 7.8 Hz, CH Ar), 7.52 (t, 1 H, J= 7.4 Hz, CH Ar), 7.40 (t, 2H, J= 7.4 Hz, CH Ar), 7.34 (d, 2H, J= 7.8 Hz, CH Ar), 7.28 (d, 2H, J= 7.8 Hz, CH Ar), 5.31 (s, 2H, CH₂), 5.16 (s, 1 H, CH), 4.46 (s, 2H, CH₂), 3.78 (s, 2H, CH₂), 1.68 (s, 3H, CH₃).
**¹³C RMN** (100 MHz, CDCl3): 211.5 (CO₂R), 191.8 (CO₂R), 177 (C-CH3), 171.2 (C-OH), 134.4 (C-Ar), 134.1 (CH Ar), 133.5 (C-Ar), 133.4 (C- Ar), 132.0 (CH Ar), 129.9 (CH Ar), 129.7 (C-Ar), 128.9 (CH Ar), 72.9 (CH-O), 66.5 (CH₂O), 51 1 (C-CH₃), 42.7 (CH₂), 40.6 (CH₂), 20.1 (CH₃).

### Example 9: Synthesis of Acetic acid 4-methyl-5-oxo-2-(3,7,11-trimethyl-dodeca-2,6,10-trienyl)-2,5-dihydro-furan-3-yl ester

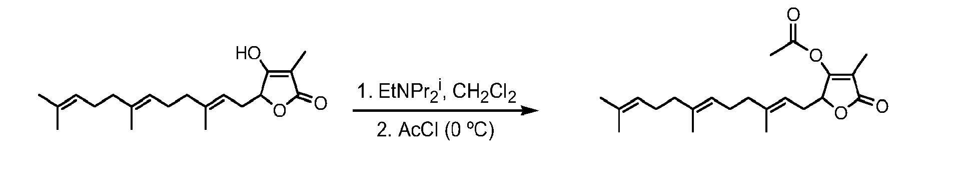

N,N-diisopropylethylamine (0.47 g, 3.65 mmol) was added drpwise to a stirred solution of 4-hydroxy-3-methyl-5-(3,7,11-trimethyl-dodeca-2,6,10-trienyl)-5H-furan-2-one (1 g, 3.14 mmol) in anhydrous CH2Cl2 (20 mL) under nitrogen atmosphere. The reaction mixture was next stirred at room temperature during 40 min. Then acetyl chloride (0.34 g, 4.30 mmol) was added dropwise to the mixture at 0°C. The mixture was stirred for 3 hours at room temperature. The solvent was evaporated in vacuum. and ethyl acetate (75 mL) and water (75 mL) were added to the resulting mixture. The organic layer was next washed with brine (75 mL), dried with sodium sulphate, filtered and concentrated in vacuum. The residue was finally purified by column chromatography using *n*-hexane/AcOEt (3:1) to give 0.66 g (58 %) of the aimed product as yellow oil.
**¹H RMN** (400 MHz, MeOD): 5.25 (m, 1 H, CH), 5.05 (ddt, 3H, CH), 2.62 (m, 1 H, CH₂), 2.36 (m, 1 H, CH₂), 2.30 (s, 3H, CH₃), 2.12-2.05 (m, 4H, CH₂), 2.0-1.90 (m, 4H, CH₂), 1. 71 (s, 3H, CH3), 1.66 (s, 3H, CH3), 1.62 (s, 3H, CH3), 1.58 (s, 6H, CH₃).
**¹³C RMN** (100 MHz, MeOD): 174.6 (CO), 167.8 (CO), 167.4 (CO), 141.6 (C-OH), 136.2 (C-CH₃), 132.1 (C-CH₃), 125.4 (CH), 125.1 (CH), 117.1 (CH), 113.7 (C-CH₃), 79.8 (CH), 40.9 (CH₂), 30.7 (CH₂), 27.8 (CH₂), 27.6 (CH₂), 25.9 (CH₃), 20. 4(CH₃), 17.8 (CH₃), 16.4 (CH₃), 16.1 (CH₃), 7.6 (CH₃).

### Example 10: Synthesis of 4-Hydroxy-3-methyl-5H-thiophen-2-one

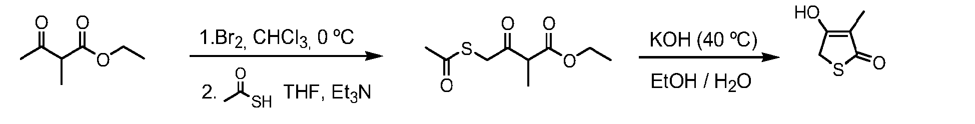

Bromine (5.55 g, 35.3 mmol) was added to a solution of 2- Methyl-3-oxo-butyric acid ethyl ester (5 g, 34.7 mmol) in 50 mL of Chloroform at 0°C. The reaction mixture was then stirred 15 hours at room temperature. The solvent was then evaporated in vacuum and the resulting mixture (4 g) was used in the next step without further purification. THF (50 mL),Triethylamine (3.64 g, 36 mmol) and thiolacetic acid (2.74 g, 36 mmol) were added to the mixture under nitrogen atmosphere and stirred at room temperature for 10 h. THF was next evaporated and CH₂Cl₂ (50 mL) and water (50 mL) were added to the resulting mixture. The organic layer was then washed with brine (50 mL), dried with sodium sulphate and concentrated in vacuum. The residue was finally purified by column chromatography using *n*-hexane/AcOEt (7:1) to give 2.25 g (54 %) of 4-Acetylsulfanyl-2-methyl-3-oxo-butyric acid ethyl ester as orange oil.
**¹H RMN** (400 MHz, CDCl₃): 4.2 (q, 2H, J= 7.2 Hz, CH₂), 3.90 (q, 2H, J= 16.6 Hz, CH₂), 3.73 (q, 1 H, J=7.1 Hz, CH), 2.38 (s, 3H, CH₃), 1.38 (d, 3H, J=7.1 Hz, CH₃), 1.28 (t, 1 H, J=7.1 Hz, CH₃).
**¹³C RMN** (100 MHz, CDCl₃): 200.1 (CO), 194.1 (CO), 170.0 (CO), 61.9 (CH₂), 52.1 (CH), 38.2 (CH₂), 30.1 (CH₃), 14,2 (CH₃), 12.5 (CH₃).

10 mL of a potassium hydroxide solution (0.84 g, 15 mmol) in water were added to a solution of 4-Acetylsulfanyl-2-methyl-3-oxo-butyric acid ethyl ester (1.70 g, 7.80 mmol) in ethanol (20 mL). The mixture was next stirred at 40 °C for 24 hours. Ethanol was evaporated in vacuum and ethyl acetate (50 mL) and water (50 mL) were added to the solution. The reaction mixture was next acidified to pH=5 with HCl 1 N. The organic layer was then washed with brine (50 mL), dried with sodium sulphate and concentrated in vacuum. The residue was finally purified by column chromatography using *n*-hexane/AcOEt/AcOH (10:10:0.5) to give 0.40 g (42 %) of the aimed product as a red solid.
**¹H RMN** (400 MHz, CD₃COCD₃): 3.90 (s, 2H, CH₂), 1.63 (s, 3H, CH₃).
**¹³C RMN** (100 MHz, CD₃COCD₃): 195.1 (CO), 175.2 (C-0H), 111.2 (C-CH₃), 32.1 (CH₂), 7.2 (CH₃).

### Example 11: Synthesis of 4-Hydroxy-3-methyl-5-(3,7,1 1 -trimethyl-dodeca-2,6,10-trienyl)-5H-thiophen-2-one

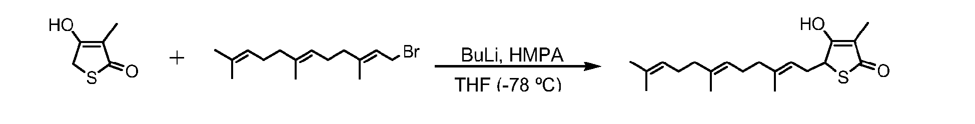

A solution of 4-Hydroxy-3-methyl-5H-thiophen-2-one (0.40 g; 3.07 mmol) and hexamethylphosphoramide (1.71 g, 9.54 mmol) in anhydrous THF (50 mL) under nitrogen atmosphere was cooled at -78 °C. Then *n*-butyllithium 1.6M in hexane (4.34 mL; 6.77 mmol) was added dropwise to the solution. The resulting mixture was stirred at -78 °C for 2 h. Then farnesyl bromide (0.96 g, 3.38 mmol) was added to the solution at -78 °C. Stirring was kept under these conditions for 1 hour, and then it was allowed to warm to room temperature and stirred 15 h. The reaction mixture was next diluted with ethyl acetate (200 mL). It was then acidized with HCl 1 N until the pH became 5. The organic layer was washed with water (200 mL) and brine (200 mL), dried with sodium sulphate, filtered and concentrated in vacuum. The residue was finally purified by column chromatography using *n*-hexane/AcOEt/AcOH (15:10:0.5) to give 0,340 g (30%) of the aimed product as yellow oil.
**¹H RMN** (400 MHz, MeOD): 5.1 (m, 3H, CH), 4.19 (dd, 1 H, J= 2.7 y 8.7 Hz, CH), 2.87 (m, 1 H, CH₂), 2.46 (m, 1 H, CH₂), 2.12-1.93 (m, 8H, CH₂), 1.66(s, 9H, 3CH₃), 1.59 (s, 6H, 2CH₃).
**¹³C RMN** (100 MHz, MeOD): 208.8 (CO), 178.5 (C-0H), 138.9 (C-CH3), 134.9 (C-CH3), 130.8 (C-CH3), 124.2 (CH), 123.9 (CH), 119.4 (CH), 110.8 (C-CH₃), 49.4 (CH), 39.6 (CH₂), 39.5 (CH), 31.0 (CH₂), 29.4 (CH₃), 26.6 (CH₂), 26.2 (CH₂), 24.7 (CH₂), 16.5 (CH₃), 15.4 (CH₃), 14.9 (CH₃), 6.3 (CH₃).

### Example 12: Synthesis of 5-(3,7-Dimethyl-octa-2,6-dienyl)-4-hydroxy-3-methyl-5H-thiophen-2-one

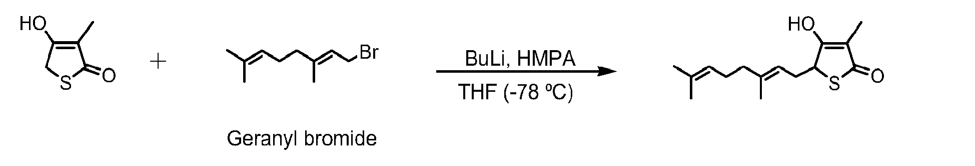

A solution of 4-Hydroxy-3-methyl-5H-thiophen-2-one (0.50 g; 3.85 mmol) and hexamethylphosphoramide (2.14 g, 11.9 mmol) in anhydrous THF (50 mL) under nitrogen atmosphere was cooled at -78 °C. Then *n*-butyllithium 1.6M in hexane (5.42 mL, 8.46 mmol) was added dropwise to the solution. The resulting mixture was stirred at -78 °C for 2 h. Then geranyl bromide (0.92 g, 4.23 mmol) was added to the solution at -78 °C. Stirring was kept under these conditions for 1 hour, and then the mixture was allowed to warm to room temperature and stirred 15 h. The reaction mixture was diluted with ethyl acetate (200 mL). It was then acidized with HCl 1 N until the pH became 5. The organic layer was washed with water (200 mL) and brine (200 mL), dried with sodium sulphate, filtered and concentrated in vacuum. The residue was finally purified by column chromatography using *n*-hexane/AcOEt/AcOH (15:10:0.5) to give 0,340 g (52%) of the aimed product as orange oil.
**¹H RMN** (400 MHz, CDCl₃): 5.24 (m, 1 H, CH), 5.05 (m, 1 H, CH), 4.14 (t, 1 H, J=7.0 Hz, CH), 2.81-2.74 (m, 1 H, CH₂), 2.55-2.48 (m, 1 H, CH₂), 2.10-1.96 (m, 8H, CH₂), 1.73 (s, 3H, CH₃), 1.68 (s, 3H, CH₃), 1.67 (s, 3H, CH₃), 1.61(s, 3H, CH₃).
**¹³C RMN** (100 MHz, CDCl₃): 179.7 (CO), 176.2 (C-O), 141.3 (C-CH₃), 133.0 (C-CH₃), 123.8 (CH), 119.8 (CH), 112.6 (C-CH₃), 48.5 (CH), 39.8 (CH₂), 31.9 (CH₂), 26.4 (CH₃), 25.9 (CH₂), 17.9 (CH₃), 16.7 (CH₃), 7.7 (CH₃).

### Example 13: Synthesis of 4-hydroxy-3-methyl-5H-furan-2-one.

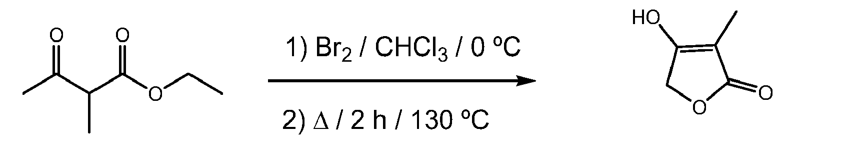

A solution of bromine (11.60 g, 71.00 mmol) in chloroform (20 ml) was added dropwise to a solution of ethyl 2-methylacetoacetate (10.00 g, 69.00 mmol) in chloroform (75 ml) at 0°C. After addition, stirring was kept for 2 hours at room temperature. The solvent was evaporated to dryness and the residue was heated at 130°C for 2 hours. A crude solid was obtained which was purified by silica gel chromatography (CH₂Cl₂ / MeOH, 12/1) to give 5.50 g (70%) of the aimed productas a white solid.
TLC (UV 254 nm): AcOEt / n-hexane / acetic acid, 5 / 10 / 0,5 Rf=0,1.
**¹H RMN** (400 MHz, MeOD): 1.6 (t, 3H, J=1.17 Hz, CH₃); 4.6 (q, 2H, J= 1.17 Hz, CH₂) allylic coupling (AB system).
**¹³C RMN** (100 MHz, MeOD): 5.8 (CH₃); 68.3 (CH₂); 96.7 (3-C(Me)=); 175.0 (C-OH); 179.1 (C=O).

### Example 14: Synthesis of 4-(2(S)-Methoxymethyl-pyrrolidin-1-yl)-3-methyl-5H-furan-2-one.

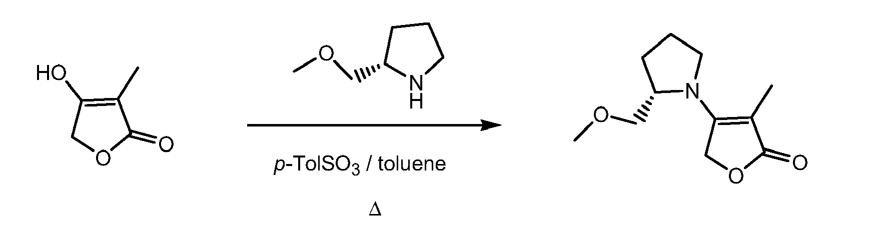

*p*-Toluenesulphonic acid monohydrate (15.00 mg, 0.08 mmol) and then (S)-2-methoxymethyl)pyrrolidine (100.00 mg, 0.87 mmol) were added into a mixture of 4-hydroxy-3-methyl-5H-furan-2-one (90.00 mg, 0.80 mmol) in dry toluene (5 ml). The reaction mixture was stirred wildly at reflux temperature (130°C) and it became a brown transparent solution. Stirring was kept at 130°C overnight. The solvent was evaporated in vacuo and the residue was purified by radial chromatography using AcOEt/MeOH (from 100/0 to 100/10 in a 2% polarity increment) to yield 136.70 mg (81%) of 4-(2(*S*)-Methoxymethyl-pyrrolidin-1-yl)-3-methyl-5H-furan-2-one as a yellow oil.
**¹H-RMN** (400 MHz, CDCl₃): 4.89 (d, 1 H, J=15 Hz, 5-CH₂ furanone); 4.53 (d, 1 H, J=15 Hz, 5-CH₂ furanone); 3.97 (m, 1 H, 2-CH pyrrolidinyl); 3.57 (m, 1 H, 5-CH₂ pyrrolodinyl); 3.42 (m, 1 H, 5-CH₂ pyrrolodinyl); 3.32 (m and s, 4H, 2-CH₂-OMe pyrrolidinyl and O-CH₃ pyrrolodinyl); 3.25 (m, 1 H, 2-CH₂-OMe pyrrolidinyl); 2.01-1.93 (m, 4H, 3-CH₂ pyrrolodinyl, 4-CH₂ pyrrolodinyl) 1.92 (s, 3H, CH₃ furanone).
**¹³C-RMN** (100 MHz, CDCl₃): 177.17 (2-CO furanone); 160.31 (4-C=N furanone); 88.70 (3-C(Me)= furanone); 74.47 (2-CH₂-Ome, pyrrolidinyl); 66.64 (5-CH₂ furanone); 59.29 (O-CH₃ pyrrolidinyl); 58.77 (2-CH pyirrolidinyl); 48.66 (5-CH₂ pyrrolidinyl); 28.01 (3-CH₂ pyrrolidinyl); 22.92 (4-CH₂, pyrrolidinyl); 8.98 (3-CH₃ furanone).

### Example 15: Synthesis of 4-(2(R)-Methoxymethyl-pyrrolidin-1-yl)-3-methyl-5-(3,7,11-trimethyl-dodeca-2,6,10-trienyl)-5H-furan-2-one.

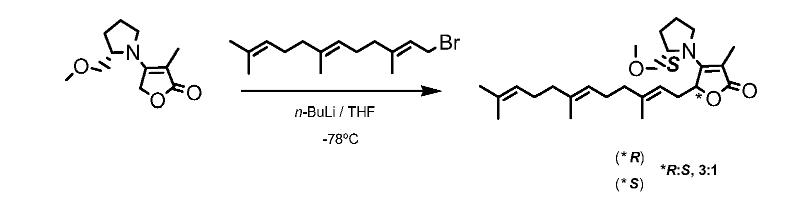

A solution of 4-(2(*R*)-Methoxymethyl-pyrrolidin-1-yl)-3-methyl-5*H*-furan-2-one (106.60 mg; 0.50 mmol) in dry THF (5 ml), under inert atmosphere, was cooled at - 75°C. A solution of *n*-butyllithium 1.6M in hexanes (347.0 □I; 0.55 mmol) was added dropwise to the former solution. Then, farnesyl bromide (150.0 □I; 0.55 mmol) was added into de solution at -75°C and stirring was kept overnight and allowed to warm to room temperature. The reaction mixture was hydrolyzed with water (10 ml) and diluted with AcOEt (15 ml). The organic layer was separated and washed with brine (1 x 10 ml), dried with sodium sulfate, filtered and concentrated in vacuum to give a crude yellow oil. The residue was purified by silica gel chromatography (Biotage flash master) using *n*-hexane/AcOEt gradient (from 100/0 to 0/100). Fractions with products were gathered and evaporated in vacuo to be purified by radial chromatography using n-hexane/AcOEt gradient (from 5/1 to 1/2 in a 2% polarity increment) to yield 80.53 mg (39%) of 4-(2(S)-Methoxymethyl-pyrrolidin-1-yl)-3-methyl-5-(3,7,11-trimethyl-dodeca-2,6,10-trienyl)-5H-furan-2-one as a yellow oil (diastereomeric mixture **R:S*, 3:1). Both diasteromers were identified in fractions enriched in each diasteromer (the first from a mixture **R*:*S*, 97:3; and .the latter from a mixture **R*:*S,* 2:1).
TLC (UV 254 nm): AcOEt / n-hexane, 1 / 2;
Rf (first compound)=0,5; Rf (second compound)=0,45.
**¹H-RMN** (400 MHz, CDCl₃) 4-(2(S)-Methoxymethyl-pyrrolidin-1-yl)-3-methyl-5-(3,7,11-trimethyl-dodeca-2,6,10-trienyl)-5H(R)-furan-2-one: 5.15 (dt, 1H, J=1 and 7 Hz, 2-CH dodecatrienyl); 5.09 (m, 2H, 6-CH y 10-CH dodecatrienyl); 4.78 (dd, 1 H, J=2 and 7 Hz, 5-CH furanone); 4.22 (m, 1 H, 2-CH pyrrolidinyl); 3.58 (m, 1 H, 5-CH₂ pyrrolidinyl); 3.38 (m, 1 H, 5-CH₂ pyrrolodinyl); 3.36 (dd, 1 H, J=4 and 9 Hz, CH₂-OMe pyrrolidinyl); 3.33 (s, 3H, O-CH₃ pyrrolodinyl); 3.24 (dd, 1 H, J=4 y 9 Hz, CH₂-OMe pyrrolidinyl); 2.63 (ddd, 1H, J=2, 7 and 15 Hz, 1-CH₂ dodecatrienyl); 2.23 (dt, 1H, J=7, 7 and 15 Hz, 1-CH₂ dodecatrienyl); 2.09-1.90 (m, 12H, 3-CH₂ pyrrolodinyl, 4-CH₂ pyrrolodinyl, 4-CH₂ dodecatrienyl, 5-CH₂ dodecatrienyl, 8-CH₂ dodecatrienyl, 9-CH₂ dodecatrienyl); 1.92 (s, 3H, CH₃ furanone); 1.68 ( s, 3H, 11-CH₃ dodecatrienyl); 1.62 (s, 3H, 3-CH₃ dodecatrienyl); 1.60 (s, 3H, 12-CH₃ dodecatrienyl); 1.59 (s , 3H, 7-CH₃ dodecatrienyl).
**¹³C-RMN** (100 MHz, CDCl₃) 4-(2(*S*)-Methoxymethyl-pyrrolidin-1-yl)-3-methyl-5-(3,7,11-trimethyl-dodeca-2,6,10-trienyl)-5H(R)-furan-2-one: 176.00 (2-CO furanone); 162.55 (4-C=N furanone); 139.10 (3-C dodecatrienyl); 135.18 (7-C dodecatrienyl); 131.27 (11-C dodecatrienyl); 124.34 (10-CH dodecatrienyl); 123.93 (6-CH dodecatrienyl); 117.20 (2-CH dodecatrienyl); 91.44 (3-C(Me)= furanone); 77.43 (5-CH furanone); 74.54 (2-CH₂-Ome, pyrrolidinyl); 59.36 (5-CH₂ pyrrolidinyl); 58.21 (2-CH pyrrolidinyl); 49.46 (O-CH₃ pyrrolidinyl); 39.83, 39.70 (3-CH₂, 4-CH₂ pyrrolidinyl); 32.13 (1-CH₂ dodecatrienyl); 27.92 (9-CH₂ dodecatrienyl); 26.75, 26.72 (4-CH₂, 8-CH₂ dodecatrienyl); 25.68 (11-CH₃ dodecatrienyl); 23.64 (5-CH₂ dodecatrienyl); 17.68 (3-CH₃ dodecatrienyl); 16.45 (12-CH₃ dodecatrienyl); 15.98 (7-CH₃ dodecatrienyl); 9,34 (3-CH₃ furanone).

| | |
|---|---|
| **NOE**: | 5-H furanone (radiated proton) - 2-H pyrrolidinyl 2.35% |
| | 2-H pyrrolidinyl (radiated proton) - 5-H furanone 0.90% |

**¹H-RMN** (400 MHz, CDCl₃) 4-(2(S)-Methoxymethyl-pyrrolidin-1-yl)-3-methyl-5-(3,7,11-trimethyl-dodeca-2,6,10-trienyl)-5H(S)-furan-2-one: 5.15 (dt, 1 H, J=1 and 7 Hz, 2-CH dodecatrienyl); 5.09 (m, 2H, 6-CH, 10-CH dodecatrienyl); 4.73 (dd, 1 H, J=2 and 7 Hz, 5-CH furanone); 4.11 (m, 1 H, 2-CH pyrrolidinyl); 3.47 (m, 2H, 5-CH₂ pyrrolidinyl); 3.39 (dd, 1 H, J=3 and 6 Hz, CH₂-OMe pyrrolidinyl); 3,33 (s, 3H, O-CH₃ pyrrolodinyl); 3.21 (dd, 1 H, J=9 and 17 Hz, CH₂-OMe pyrrolidinyl); 2.76 (ddd, 1 H, J=2, 7 and 15 Hz, 1-CH₂ dodecatrienyl); 2.27 (m, 1 H, 1-CH₂ dodecatrienyl); 2.23 (dt, 1 H, J=7, 7 and 15 Hz, 1-CH₂ dodecatrienyl); 2.09-1.90 (m, 12H, 3-CH₂ pyrrolodinyl, 4-CH₂ pyrrolodinyl, 4-CH₂ dodecatrienyl, 5-CH₂ dodecatrienyl, 8-CH₂ dodecatrienyl, 9-CH₂ dodecatrienyl); 1.92 (s, 3H, CH₃ furanone); 1.68 (s, 3H, 11-CH₃ dodecatrienyl); 1.62 (s, 3H, 3-CH₃); 1.60 (s, 3H, 12-CH₃ dodecatrienyl); 1.59 (s , 3H, 7-CH₃ dodecatrienyl).
**¹³C-RMN** (100 MHz, CDCl₃) 4-(2(S)-Methoxymethyl-pyrrolidin-1-yl)-3-methyl-5-(3,7,11-trimethyl-dodeca-2,6,10-trienyl)-5H(S)-furan-2-one: 176.00 (2-CO furanone); 162.08 (4-C=N furanone); 139.27 (3-C dodecatrienyl); 135.18 (7-C farnesilo); 131.27 (11-C dodecatrienyl); 124.33 (10-CH dodecatrienyl); 123.93 (6-CH dodecatrienyl); 117.07 (2-CH dodecatrienyl); 89.98 (3-C(Me)= furanone); 77.67 (5-CH furanone); 73.15 (2-CH₂-Ome, pyrrolidinyl); 59.42 (5-CH₂ pyrrolidinyl); 58.72 (2-CH pyrrolidinyl); 49.26 (O-CH₃ pyrrolidinyl); 39.82, 39.0 (3-CH₂, 4-CH₂ pyrrolidinyl); 31.72 (1-CH₂ dodecatrienyl); 28.30 (9-CH₂ dodecatrienyl); 26.75, 26.71 (4-CH₂, 8-CH₂ dodecatrienyl); 25.68 (11-CH₃ dodecatrienyl); 22.37 (5-CH₂ dodecatrienyl); 17.67 (3-CH₃ dodecatrienyl); 16.49 (12-CH₃ dodecatrienyl); 15.98 (7-CH₃ dodecatrienyl); 9.34 (3-CH₃ furanone).

| | |
|---|---|
| **NOE** : | 5-H furanone (radiated proton) - 2-H pyrrolidinyl 1.18% |
| | 2-H pyrrolidinyl (radiated proton) - 5-H furanone 0.0% |

### Example 16: Synthesis of 4-methoxymethoxy-3-methyl-5H-furan-2-one.

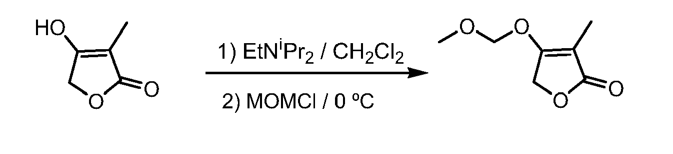

N,N-diisopropylethylamine (3.45 ml, 198.00 mmol) was added dropwise to a solution of 4-hydroxy-3-methyl-5H-furan-2-one (2.00 g, 175.00 mmol) in dry dichloromethane (20 ml), at room temperature and under inert atmosphere. The stirring was kept in those conditions for 30 minutes. The solution was cooled at 0°C and chloromethylmethyl ether (1.85 ml, 243.00 mmol) was added dropwise. After said addition, stirring was kept at room temperature for 3 hours. The solvent was next evaporated in vacuum and the residue was extracted with AcOEt (75 ml) and washed with water (1 x 75 ml). The organic layer was separated and washed with brine (1 x 100 ml), dried with sodium sulfate, filtered and evaporated till dryness. The residue was finally purified by silica gel chromatography (n-hexane / AcOEt, 1/1) to give 1.75 g (64%) of 4-methoxymethoxy-3-methyl-5H-furan-2-one as a yellow oil.
**¹H RMN** (400 MHz, MeOD): 5.09 (s, 2H, CH₂ MOM); 4.67 (s, 2H, 5-CH₂ furanone); 3.46 (s, 3H, CH₃ MOM); 1.75 (s, 3H, CH₃ furanone).
**¹³C RMN** (100 MHz, MeOD): 175.04 (CO furanone); 170.08 (O-C=furanone); 101.64 (=C-Me furanone); 95.23 (CH₂ MOM); 66.35 (CH₂ furanone); 57.16 (CH₃ MOM); 6.96 (CH₃ furanone).

### Example 17: Synthesis of 4-hydroxy-3-methyl-5-(3,7,11-trimethyl-dodeca-2,6,10-trienyl)-5H-furan-2-one.

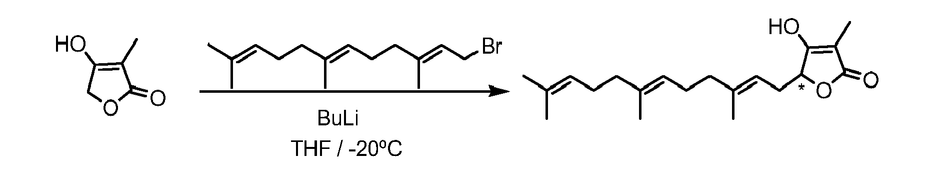

A solution of 4-hydroxy-3-methyl-5H-furan-2-one (1.00 g; 8.80 mmol) in dry THF (150 ml), under inert atmosphere, was cooled at -20°C. A solution of *n*-butyllithium 1.6M in hexanes (12 ml; 19.20 mmol) was added dropwise to the former solution. The resulting mixture was strongly stirred at -20°C for 30 minutes, and a white solid was formed. Then, farnesyl bromide (220.0 µl; 8.10 mmol) was added into the solution at -20°C; the solution getting a yellow colour. Stirring was kept under these conditions for 1 hour, and then it was allowed to warm to room temperature for 1 hour more. The reaction mixture was next diluted with dichloromethane (200 ml) and afterwards hydrolyzed with water (150 ml). A white emulsion was obtained in the aqueous layer, at pH 9. The whole mixture was then acidized with HCl 37% up to pH 1. The emulsion was cleared up, and the product was extracted in dichloromethane (2x 200 ml). Next, the combined organic extracts were washed with brine (1 x 300 ml), dried with sodium sulfate, filtered and concentrated in vacuum to give a crude yellow oil. The residue was finally purified by silica gel chromatography (Biotage flash master) using n-hexane/AcOEt gradient (from 100/0 to 100/20 in a 2% polarity increment) to yield 1,15 g (45%) of the aimed compound as a yellow oil (enantiomeric mixture).
TLC (UV 254 nm): AcOEt / n-hexane / acetic acid, 5 / 10 / 0,5. Rf=0,4
**¹H RMN** (400 MHz, MeOD): 5.10 (m, 3H, CH); 4.73 (m, 1 H, CH); 2.65 (m, 1 H, CH₂); 2.37 (m, 1H, CH₂); 2.15-2.03(m, 4H, CH₂); 2.00-1.94 (m, 4H, CH₂); 1. 65,1.64, 1.63, 1.60(s, 3H, CH₃); 1.58 (s, 3H, CH₃).
**¹³C RMN** (100 MHz, MeOD): 177.03 (CO); 175.36 (C-OH); 139.67, 134.95, 130.87 (C); 124.30, 124.04, 116.44 (CH); 96.29 (C-Me); 78.27 (CH); 39.75, 39.66 (CH₂); 26.66, 26.53, 24.73 (CH₂); 16.61, 15.34, 15.33, 14.92, 4.75 (CH₃).

### Example 18: Synthesis of 3-Methyl-4-proaoxy-5-(3,7,11-trimethyl-dodeca-2,6,10-trienyl)-5H-furan-2-one (NP-05910) and 4-Methyl-5-propoxy-2-(3,7,11-trimethyl-dodeca-2,6,10-trienyl)-furan-3-one.

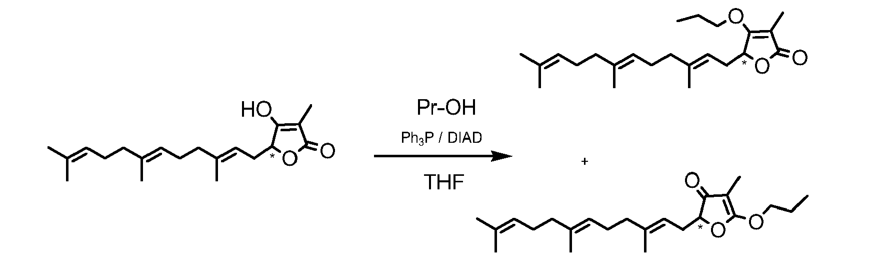

Triphenylphosphine (165.00 mg, 0.63 mmol), diisopropyl azodicarboxylate (DIAD) (124.0 µl, 0.63 mmol) and propanol (47.0 µl, 0.63 mmol) were successively added to a solution of 4-hydroxy-3-methyl-5-(3,7,11-trimethyl-dodeca-2,6,10-trienyl)-5*H*-furan-2-one (200.00 mg, 0.63 mmol) in dry THF (5 ml), previously stirred under inert atmosphere at room temperature for 5 minutes,. The reaction mixture was stirred at room temperature overnight. The solvent was then evaporated in vacuum and the residue was purified by radial chromatografy using *n*-hexane/AcOEt gradient (from 100/0 to 50/50) to yield 100 mg of a mixture of the described products. That mixture was finally purified in a second round by radial chromatografy using *n*-hexane/AcOEt gradient (from 100/0 to 50/50) to yield 72.6 mg (26%) of 3-Methyl-4-propoxy-5-(3,7,11-trimethyl-dodeca-2,6,10-trienyl)-5H-furan-2-one as a colourless oil (enantiomeric mixture), and 14.0 mg (6%) of 4-Methyl-5-propoxy-2-(3,7,11-trimethyl-dodeca-2,6,10-trienyl)-furan-3-one as a colourless oil (enantiomeric mixture).
TLC (UV 254 nm): AcOEt / n-hexane / acetic acid, 5 / 15 / 0,5,
Rf (first compound)=0,6; Rf(second compound)=0,5.
**¹H RMN** (400 MHz, CDCl₃) 3-Methyl-4-propoxy-5-(3,7,11-trimethyl-dodeca-2,6,10-trienyl)-5H-furan-2-one: 5.10 (m, 3H, 2-CH dodecatrienyl, 6-CH dodecatrienyl, 10-CH dodecatrienyl); 4.61 (m, 1 H, 5-CH furanone); 4.27 (m, 2H, J= 6, 5, 9 and 16 Hz, 1'-CH₂ propyl); 2.61 (ddd, 1 H, J=4, 7 and 15 Hz, 1-CH₂ dodecatrienyl); 2.31 (dd, 1 H, J=7, 7 and 15 Hz, 1-CH₂ dodecatrienyl); 2.09-1.95 (m, 8H, 4-CH₂, 5-CH₂, 8-CH₂, 9-CH₂ dodecatrienyl); 1.93 (s, 3H, 3-CH₃ furanone); 1.76 (sex., 1 H, 2'-CH₂ propyl); 1.67, 1.62, 1.60, 1.58 (s, s, s, s, 3H, 3H, 3H, 3H, 12-CH₃, 11-CH₃, 7-CH₃, 3-CH₃ dodecatrienyl); 1.01 (t, 3H, J=7 Hz, 3'-CH₃ propyl).
**¹³C RMN** (100 MHz, CDCl₃) 3-Methyl-4-propoxy-5-(3,7,11-trimethyl-dodeca-2,6,10-trienyl)-5H-furan-2-one: 175.11 (CO furanone); 172.63 (O-C=furanone); 139.55 (3-C dodecatrienyl); 135.18 (7-C dodecatrienyl); 131.28 (11-C dodecatrienyl); 124.34, 123.92 (6-CH=, 10-CH= dodecatrienyl); 116.48 (2-CH= dodecatrienyl); 97.29 (3-C= furanone); 77.62 (5-CH furanone); 72.78 (1'-ch₂ propyl); 39.76, 39.71 (5-CH₂, 9-CH₂ dodecatrienyl); 30.44 (1-CH₂ dodecatrienyl); 26.75, 26.67 (4-CH₂, 8-CH₂ dodecatrienyl); 25.68, 17.67, 16.35, 15.97 (12-CH₃, 11-CH₃, 7-CH₃, 3-CH₃ dodecatrienyl); 22.99 (2'-ch₂ propyl); 10.06 (3'-ch₃ propyl); 8.54 (3-CH₃ furanone).
**¹H RMN** (400 MHz, CDCl₃) 4-Methyl-5-propoxy-2-(3,7,11-trimethyl-dodeca-2,6,10-trienyl)-furan-3-one: 5.14 (t, 1H, J= 7Hz, 2-CH dodecatrienyl); 5.09 (m, 2H, 6-CH, 10-CH dodecatrienyl); 4.53 (dd, 1 H, J= 4 and 7 Hz, 2-CH furanone); 4.28 (t, 2H, J= 7Hz, 1-CH₂ propyl); 2.71 (ddd, 1H, J=4, 7 and 15 Hz, 1-CH₂ dodecatrienyl); 2.39 (dt, 1H, J=7, 7 and 15 Hz, 1-CH₂ dodecatrienyl); 2.10-1.91 (m, 8H, 4-CH₂, 5-CH₂, 8-CH₂, 9-CH₂ dodecatrienyl); 1.78 (sex., 2H, J= 7Hz, 2-CH₂ propyl); 1.67, 1.59 (s, s, 3H, 3-H, 12-CH₃, 11-CH₃ dodecatrienyl); 1.63 (s, 3H, 3-CH₃ dodecatrienyl); 1.59 (s, 6H, 7-CH₃ dodecatrienyl, 4-CH₃ furanone); 1.01 (t , 3H, 3-CH₃ propyl).
**¹³C RMN** (100 MHz, CDCl₃) 4-Methyl-5-propoxy-2-(3,7,11-trimethyl-dodeca-2,6,10-trienyl)-furan-3-one: 198.16 (3-C=O furanone); 180.47 (5-C(O)₂= furanone); 139.33 (3-C dodecatrienyl); 135.18 (7-C dodecatrienyl); 131.31 (11-C dodecatrienyl); 124.29, 123.86 (6-CH, 10-CH dodecatrienyl); 117.06 (2-CH dodecatrienyl); 89.01 (4-C(Me)= furanone); 86.65 (2-CH furanone); 71.02 (1-CH₂ propyl); 39.98, 39.95 (4-CH₂, 8-CH₂ dodecatrienyl); 30.04 (1-CH₂ dodecatrienyl); 26.98, 26.81 (5-CH₂, 9-CH₂ dodecatrienyl); 25.91 (2-CH₂ propyl); 22.75, 17.90 (12-CH₃, 11-CH₃ dodecatrienyl); 16.56, 16.20 (7-CH₃, 3-CH₃ dodecatrienyl); 10.29 (3-CH₃ propyl); 4.17 (4-CH₃ furanone).

### Example 19: Synthesis of 4-Methoxymethoxy-3-methyl-5-(3,7,11-trimethyl-dodeca-2,6,10-trienyl)-5H-furan-2-one (NP-0525).

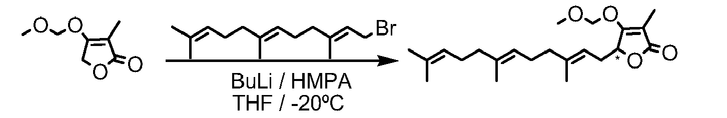

A solution of *n*-butyllithium 1.6M in hexanes (8.8 ml; 14.00 mmol) was added dropwise to a solution of 4-methoxymethoxy-3-methyl-5H-furan-2-one (2.00 g; 12.60 mmol) and hexamethylphosphoramide (3.4 ml, 19.80 mmol) in dry THF (20 ml), at -78°C. The resulting mixture was stirred at -78°C for 30 minutes. The mixture gradually became yellow. Then, farnesyl bromide (3.8 ml; 14.00 mmol) was added dropwise and the mixture was allowed to warm to room temperature for 2 hours. The reaction mixture was next diluted with dichloromethane (40 ml) and hydrolyzed with water (30 ml). The product was extracted in dichloromethane (2x 40 ml) and the combined organic extracts were washed with brine (1 x 60 ml), dried with sodium sulfate, filtered and concentrated in vacum to give a crude yellow oil. The residue finally was purified by silica gel chromatography (Biotage flash master) using *n*-hexane/AcOEt gradient (from 100/0 to 50/50) to yield 3.3 g (72%) of the aimed compound as a yellow oil (enantiomeric mixture).
TLC (UV 254 nm): AcOEt / n-hexane / acetic acid, 5/10/0,5; Rf=0,5.
**¹H RMN** (400 MHz, CDCl₃): 5.25 (d, 1 H, J= 6 Hz, CH₂ MOM); 5.20 (d, 1 H, J= 6 Hz, CH₂ MOM); 5.09 (m, 3H, 2-CH dodecatrienyl, 6-CH dodecatrienyl, 10-CH dodecatrienyl); 4.68 (m, 1 H, 5-CH furanone); 3.51 (s, 3H, CH₃ MOM); 2.65 (ddd, 1 H, J=4, 7 and 15 Hz, 1-CH₂ dodecatrienyl); 2.32 (dt, 1H, J=7, 7 and 15 Hz, 1-CH₂ dodecatrienyl); 2.08-1.94 (m, 8H, 4-CH₂, 5-CH₂, 8-CH₂, 9-CH₂ dodecatrienyl); 1.89 (s, 3H, 3-CH₃ furanone); 1,68, 1,60 (s, s, 3H, 3H, 12-CH₃, 11-CH₃ dodecatrienyl); 1,63 (s, 3H, 3-CH₃ dodecatrienyl); 1,59 (s , 3H, 7-CH₃ dodecatrienyl).
**¹³C RMN** (100 MHz, CDCl₃): 174.7 (CO furanone); 170.4 (O-C=furanone); 139.7 (3-C dodecatrienyl); 135.2 (7-C dodecatrienyl); 131.3 (11-C dodecatrienyl); 124.3, 123.9 (6-CH, 10-CH dodecatrienyl); 116.3 (2-CH dodecatrienyl); 100.1 (=C-Me furanone); 95.0 (CH₂ MOM); 77.6 (CH furanone); 57.0 (CH₃ MOM); 39.8; 39.7 (4-CH₂, 8-CH₂ dodecatrienyl); 26.7, 26.6 (5-CH₂, 9-CH₂ dodecatrienyl); 30.5 (1-CH₂ dodecatrienyl); 25.7, 17.7 (12-CH₃, 11-CH₃ dodecatrienyl); 16.4, 16.0 (7-CH₃, 3-CH₃ dodecatrienyl); 7.9 (CH₃ furanone).

### Example 20: Synthesis of 4-methoxymethoxy-3-methyl-5-(3,7,11-trimethyl-6,7-epoxy-dodeca-2, 10-dienyl)-5H-furan-2-one (NP-0591) and 4-methoxymethoxy-3-methyl-5-(3,7,11-trimethyl-10,11-epoxy-dodeca-2, 6-dienyl)-5H-furan-2-one (NP-0592).

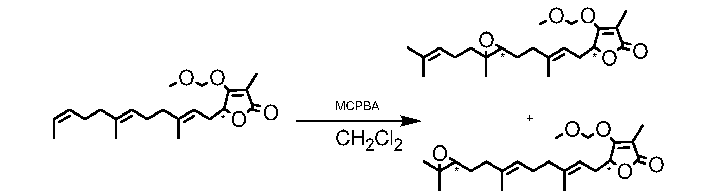

A solution of 4-methoxymethoxy-3-methyl-5-(3,7,11-trimethyl-dodeca-2,6,10-trienyl)-5H-furan-2-one (0.56 g, 1.50 mmol) in anhydrous dichloromethane (5 ml) was added to a solution of 3-chloroperbenzoic acid (MCPBA) (0.29 g, 1.70 mmol) in anhydrous dichloromethane (5 ml) at 0°C under inert atmosphere. Stirring was kept under those conditions for 4 hours. As far as the starting material had not completely reacted, more solid MCPBA (0.16 g, 0.9 mmol) was added into the reaction mixture at 0°C. Stirring was kept overnight and the solution was allowed to warm to room temperature. The final reaction mixture pH was 5. Next, aqueous NaHCO₃ 10% was added until a pH of 9 was reached. The organic layer was then separated and the aqueous layer was extracted with dichloromethane. The combined organic layers were gathered and dried with sodium sulfate, filtered and concentrated in vacuum to give a crude yellow oil. The residue was purified by silica gel chromatography (Biotage flash master) using *n*-hexane/AcOEt gradient (from 100/0 to 4/1) to yield 329.00 mg of a mixture of oxyranyl compounds. Finally, a second purification by radial chromatography was performed using *n*-hexane/AcOEt gradient (from 50/1 to 50/20) to yield two products as diasteromeric mixtures: 4-methoxymethoxy-3-methyl-5-(3,7,11-trimethyl-6,7-epoxy-dodeca-2,10-dienyl)-5*H*-furan-2-one 61.20 mg (10%), and 4-methoxy-methoxy-3-methyl-5-(3,7,11-trimethyl-10,11-epoxy-dodeca-2,6-dienyl)-5*H*-furan-2-one, 79.80 mg (14%).
TLC (UV 254 nm): AcOEt / n-hexane, 1 / 2; Rf (first compound)=0,6; Rf (second compound)=0,5.
**¹H RMN** (400 MHz, CDCl₃) 4-methoxymethoxy-3-methyl-5-(3,7,11-trimethyl-6,7-epoxydodeca-2, 10-dienyl)-5H-furan-2-one: 5.24 (d, 1 H, J= 6 Hz, CH₂ MOM); 5.21 (d, 1 H, J= 6 Hz, CH₂ MOM); 5.15 (m, 2H, 2-CH dodecadienyl); 5.07 (t, J= 7 Hz, 10-CH dodecadienyl); 4.68 (m, 1 H, 5-CH furanone); 3.50 (s, 3H, CH₃ MOM); 2.66 (m, 2H, 6-CH, 1-CH₂ dodecadienyl); 2.31 (m, 1H, 1-CH₂ dodecadienyl); 2.18-1.95 (m, 4H, 4-CH₂, 8-CH₂ dodecadienyl); 1.87 (s, 3H, 3-CH₃ furanone); 1.67, 1.64, 1.60 (s, s, s, 3H, 3H, 3H, 12- CH₃, 11-CH₃, 3-CH₃ dodecadienyl); 1.63-1.55 (m, 3H, 5-CH₂, 9-ch₂ dodecadienyl); 1.40 (m, 1 H, 9-ch₂ dodecadienyl); 1.23 (s , 3H, 7-CH₃ dodecadienyl).
**¹³C RMN** (100 MHz, CDCl₃) 4-methoxymethoxy-3-methyl-5-(3,7,11-trimethyl-6,7-epoxydodeca-2, 10-dienyl)-5H-furan-2-one: 174.13 (2-CO furanone); 169.80 (4-OC= furanone); 138.41 (3-C dodecadienyl); 131.35 (11-C dodecadienyl); 123.18 (10-C dodecadienyl); 116.49, 116.38 (2-CH dodecadienyl); 99.64 (3-C(Me)= furanone); 94.59 (CH₂ MOM); 76.97, 77.00 (5-CH furanone); 62.69 (6-C dodecadienyl); 60.26, 60.23 (7-C dodecadienyl); 56.52 (CH₃ MOM); 38.28 (9-CH₂ dodecadienyl); 35.89 (8-CH₂ dodecadienyl); 30.02, 29.92 (1-ch₂ dodecadienyl); 26.91, 26.79 (5-ch₂ dodecadienyl); 25.17 (3-CH₃ dodecadienyl); 23.39 (4-CH₂ dodecadienyl); 17.17, 15.88, 15.84 (12-CH₃, 11-CH₃ dodecadienyl); 16.00 (7-CH₃ dodecadienyl); 7.28 (CH₃ furanone).
**¹H RMN** (400 MHz, CDCl₃) 4-methoxymethoxy-3-methyl-5-(3,7,11-trimethyl-10,11-epoxy-dodeca-2, 6-dienyl)-5H-furan-2-one: 5.26 (d, 1 H, J= 6 Hz, CH₂ MOM); 5.20 (d, 1 H, J= 6 Hz, CH₂ MOM); 5,12 (m, 2H, 2-CH, 6-CH dodecadienyl); 4.68 (m, 1 H, CH furanone); 3.50 (s, 3H, CH₃ MOM); 2.70 (t, 1 H, J=6 Hz, 10-CH dodecadienyl); 2.65 (ddd, 1H, J=4, 7 and 15 Hz, 1-CH₂ dodecadienyl); 2.30 (dt, 1H, J= 7 and 15 Hz, 1-CH₂ dodecadienyl); 2.19-1.97 (m, 4H, 4-CH₂ and 5-CH₂ dodecadienyl); 1.90 (s, 3H, CH₃ furanone); 1.63, 1.61 (s, s, 3H, 3H, 7-CH₃, 3-CH₃ dodecadienyl); 1.71-1.56 (m, 4H, 9-ch₂, 8-ch₂ dodecadienyl); 1.30 (s, 3H, 12-CH₃ dodecadienyl); 1.26 (s , 3H, 11-CH₃).
**¹³C RMN** (100 MHz, CDCl₃) 4-methoxymethoxy-3-methyl-5-(3,7,11-trimethyl-10,11-epoxy-dodeca-2, 6-dienyl)-5H-furan-2-one: 174.56 (2-CO furanone); 170.32 (4-OC= furanone); 139.38 (3-C dodecadienyl); 134.14 (7-C dodecadienyl); 124.28, 116.09 (6-CH, 2-CH dodecadienyl); 99.70 (3-C-Me furanone); 94.97 (CH₂ MOM); 77.43 (5-CH furanone); 64.07 (1-ch₂ dodecadienyl); 58.11 (11-C dodecadienyl); 56.70 (CH₃ MOM); 39.46 (5-ch₂ dodecadienyl); 36.14 (4-CH₂ dodecadienyl); 30.31 (10-CH dodecadienyl); 27.22 (9-CH₂ dodecadienyl); 26.42 (8-CH₂ dodecadienyl); 24.70 (12-CH₃ dodecadienyl); 18.52 (11-CH₃ dodecadienyl); 16.12, 15.89 (7-CH₃, 3-CH₃ dodecadienyl); 7.77(CH₃ furanone).

### Example 21: Synthesis of 3-Methyl-4-pyrrolidin-1-yl-5H-furan-2-one.

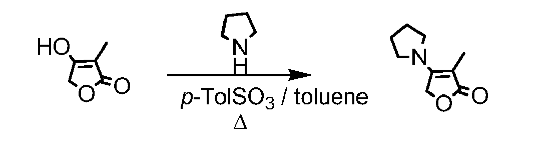

*p*-Toluenesulphonic acid monohydrate (170.0 mg, 0.90 mmol) and then pyrrolidine ( 0.81 µl, 9.70 mmol) were added into a mixture of 4-hydroxy-3-methyl-5H-furan-2-one (1.00 g, 8.80 mmol) in dry toluene (30 ml). The reaction mixture was vigorously stirred at reflux temperature (130°C) using a Dean-Stark apparatus. Stirring was kept at 130°C for 24 hours. The solvent was next evaporated in vacuum and the residue was finally purified by silica gel chromatography (Biotage flash master) using *n-*hexane/AcOEt gradient (from 100/5 to 100/50) to yield 1.31 g (89%) of the aimed compound as a light brown solid.
TLC (UV 254 nm): AcOEt / n-hexane, 3/1; Rf=0,3
**¹H RMN** (400 MHz, CDCl₃): 4.51 (s, 2H, 5-CH₂ furanone); 3.47 (m, 4H, 2-CH₂ pyrrolidinyl); 1.94 (m, 4H, 3-CH₂ pyrrolidinyl); 1.91 (s, 3-CH₃ furanone).
**¹³C RMN** (100 MHz, CDCl₃): 177.11 (CO furanone); 160.33 (4-C= furanone); 87.81 (3-C= furanone); 66.22 (5-CH₂ furanone); 48.27 (2-CH₂ pyrrolidinyl); 25.10 (3-CH₂ pyrrolidinyl); 8.49 (3-CH₃ furanone).

### Example 22: Synthesis of 4-(pyrrolidin-1-yl)-3-methyl-5-(6-cyanopentyl)-5H-furan-2-one.

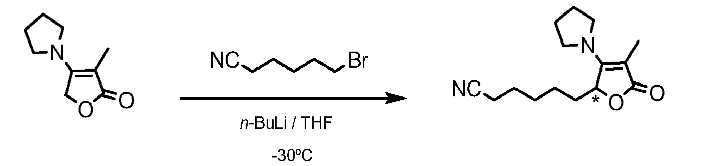

A solution of *n*-butyllithium 1.6M in hexanes (1.25 ml; 2.00 mmol) was added dropwise to a solution of 3-methyl-4-pyrrolidin-1-yl-5*H*-furan-2-one (300.0 mg; 1.80 mmol) in dry THF (10 ml), at -30°C. The resulting mixture was stirred at -30°C for 45 minutes. Then, 6-bromohexanenitrile (265 □l; 2.00 mmol) was added dropwise and the stirring was kept at -30°C for 1 hour. Afterwards, the solution was allowed to warm to room temperature for 2 hours. The reaction mixture was next diluted with dichloromethane (20 ml) and hydrolyzed with water (10 ml). The aqueous layer was then acidized up to pH 1 with HCl 10% and extracted with dichloromethane (2x 20 ml). The combined organic extracts were washed with brine (1 x 30 ml), dried with sodium sulfate, filtered and concentrated in vacuum. The residue was finally purified by silica gel chromatography (Biotage flash master) using *n*-hexane/AcOEt gradient (from 4/1 to 1/2), and the fractions containing product were gathered and purified again by radial chromatography using *n*-hexane/AcOEt gradient (from 2/1 to 5/6) to yield 278.9 mg (59%) of the aimed produc as a colourless oil (enantiomeric mixture).
TLC (UV 254 nm): AcOEt / n-hexane, 3/1; Rf=0,6
**¹H RMN** (400 MHz, CDCl₃): 4.69 (dd, 1 H, J=2 and 7 Hz, 5-CH furanone); 3.52 (m, 4H, 2-CH₂ pyrrolidinyl); 2.33 (t, 2H, J=7 Hz, 5-CH₂ cyanopentyl); 2.00, 1.86 (2m, 4H, 3-CH₂ pyrrolidinyl); 1.93, 1.51 (2m, 2H, 2-CH₂ cyanopentyl); 1.92 (s, 3H, CH₃ furanone); 1.62 (m, 2H, 4-CH₂ cyanopentyl); 1.54 - 1.40 (m, 4H, 1-CH₂, 3-CH₂ cyanopentyl).
**¹³C RMN** (100 MHz, CDCl₃): 176.08 (2-CO furanone); 163.32 (4-C= furanone); 119.60 (CN) 89.53 (3-C(Me)= furanone); 76.88 (5-CH furanone); 49.32 (2-CH₂ pyrrolidinyl); 32.73 (2-CH₂ cyanopentyl); 28.24 (1-CH₂ cyanopentyl); 25.21 (3-CH₂ pyrrolidinyl); 25.14 (4-CH₂ cyanopentyl); 23.82 (3-CH₂, cyanopentyl); 17.03 (5-CH₂ cyanopentyl); 8.98 (CH₃ furanone).

### Example 23: Synthesis of 4-hydroxy-3-methyl-5-(6-cyanopentyl)-5H-furan-2-one.

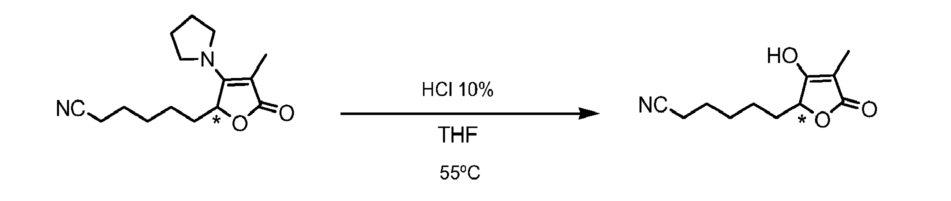

A solution of 4-(pyrrolidin-1-yl)-3-methyl-5-(6-cyanopentyl)-5*H*-furan-2-one (268.0 mg, 0.76 mmol) in THF (20 ml) was prepared and next an aqueous solution of HCI 10% (10 ml) was added thereto. The reaction mixture was next stirred at 55°C for 24 hours. Then, the mixture was concentrated to half volume in vacuum and diluted with AcOEt (20 ml). The resulting solution was next washed with water (1 x 15 ml) and the organic layer was separated. Afterwards the aqueous layer was neutralized with solid NaHCO₃ (from pH1 to pH 6) and extracted with AcOEt. The organic extracts were gathered and washed with brine (1 x 20 ml), separated and dried with sodium sulfate, and finally filtered and evaporated till dryness. The residue was next purified by radial chromatography using *n*-hexane/AcOEt gradient (from 5/1 to 0/1) and then AcOEt/MeOH gradient (from 10/1 to 1/1) to yield 16.0 mg (10%) of the aimed compound as a light yellow oil (enantiomeric mixture).
TLC (UV 254 nm): CH₂Cl₂ / MeOH, 10/0.5; Rf=0,5
**¹H RMN** (400 MHz, MeOD): 4.43 (dd, 1 H, J=3 and 7 Hz, 5-CH furanone); 2.43 (t, 2H, J=7 Hz, 5-CH₂ cyanopentyl); 1.93 (m, 1 H, 1-CH₂ cyanopentyl); 1.64 - 1.39 (m, 7H, 1-CH₂, 2-CH₂, 3-CH₂, 4-CH₂ cyanopentyl); 1.58 (s, 3H, CH₃ furanone).
**¹³C RMN** (100 MHz, MeOD): 187.87 (2-CO furanone); 182.05 (4-C(OH)= furanone);121.21 (CN) 91.26 (3-C(Me)= furanone); 81.34 (5-CH furanone); 32.67 (2-CH₂ cyanopentyl); 29.55 (1-CH₂, cyanopentyl); 26.42 (4-CH₂ cyanopentyl); 24.90 (3-CH₂ cyanopentyl); 17.24 (5-CH₂ cyanopentyl); 5.93 (CH₃ furanone).

### Biological Methods

### GSK-3 beta INHIBITION ASSAY

The GSK-3 beta activity of the compounds of formula (I) according to the present invention was determined by incubation of a mixture of recombinant human GSK-3 enzyme, a phosphate source and GSK-3 substrate in the presence and in the absence of the corresponding test compound, and by measuring the GSK-3 activity of this mixture. The compounds where tested at final concentrations of 25 and 50 µM.

Recombinant human glycogen synthase kinase 3 beta was assayed in MOPS 8 mM pH 7.3, EDTA 0.2 mM, MgCl₂ 10 mM and sodium orthovanadate 0.25 mM in the presence of 62.5 µM of Phospho-Glycogen Synthase Peptide-2 (GS-2), 0.5 µCi gamma-³³P-ATP and unlabelled ATP at a final concentration of 12.5 µM. The final assay volume was 20 µl. After incubation for 30 minutes at 30 °C, 15 µl aliquots were spotted onto P81 phosphocellulose papers. Filters were washed four times for at least 10 minutes each and counted with 1.5 ml of scintillation cocktail in a scintillation counter.

The compounds of formula (I) of the present invention where submitted to the above indicated assays, in order to determine both their GSK-3 inhibition activity and BACE activity inhibition. The results are indicated in Table I, in percentage of the respective enzyme activity.

| **Compound** **(NAME)** | **% GSK-3 Activity** | |
|---|---|---|
| | **25µM** | **50µM** |
| 5-[2-(tert-Butyl-diphenyl-silanyloxy)-ethyl]-5-methoxy-5H-furan-2-one | - | 24, 19 |
| 5-[3-(tert-Butyl-diphenyl-silanyloxy)-propyl]-5-methoxy-5H-furan-2-one | - | 7, 89 |
| 5-[4-(tert-Butyl-diphenyl-silanyloxy)-butyl]-5-methoxy-5H-furan-2-one | - | 15, 72 |
| 5-(4-hydroxybutyl)-5-methoxy-5H-furan-2-one | - | 62, 98 |
| 5-[5-(tert-Butyl-diphenyl-silanyloxy)-pentyl]-5-methoxy-5H-furan-2-one | - | 67, 65 |
| 5-[6-(tert-Butyl-diphenyl-silanyloxy)-hexyl]-5-methoxy-5H-furan-2-one | - | 8, 44 |
| 5-[4-(tert-Butyl-diphenyl-silanyloxy)-3-methyl-butyl]-4-hydroxy-3-methyl-5H-furan-2-one | 83,4 | 59,4 |
| 5-[7-(tert-Butyl-diphenyl-silanyloxy)-2,6-dimethyl-hept-2-enyl]-4-methoxy-3-methyl-5H-furan-2-one | 8,44 | 7,86 |
| 5-(6-Furan-3-yl-3-methyl-hex-2-enyl)-4-methoxy-3-methyl-5H-furan-2-one | 61 | 42 |
| 4-Hydroxy-3-naphthalen-2-ylmethyl-5H-furan-2-one | 55 | 66 |
| 3-Benzyl-4-hydroxy-5H-furan-2-one | 59 | 72 |
| 4-Hydroxy-3-methyl-5H-thiophen-2-one | 76,46 | 48,26 |
| 4-Hydroxy-3-pentyl-5H-furan-2-one | 100 | 67 |
| 4-Hydroxy-3-methyl-5-(3,7,11-trimethyl-dodeca-2,6,10-trienyl)-5H-furan-2-one | 65 | 25 |
| 4-Hydroxy-3-methyl-5-(3,7,11-trimethyl-dodeca-2,6,10-trienyl)-5H-thiophen-2-one | 9 | 5 |
| 5-(3,7-Dimethyl-octa-2,6-dienyl)-4-hydroxy-3-methyl-5H-furan-2-one | 80 | 59 |
| 4-Hydroxy-3-methyl-5-undec-10-enyl-5H-furan-2-one | 81 | 57 |
| [4-(3-Hydroxy-4-methyl-5-oxo-2,5-dihydro-furan-2-ylmethyl)-phenyl]-acetic acid 2-oxo-2-phenyl-ethyl ester | 90 | 69 |
| 4-Methoxymethoxy-3-methyl-5-(3,7,11-trimethyl-dodeca-2,6,10-trienyl)-5H-furan-2-one | 61 | 18,80 |
| 5-(4-Hydroxy-3-methyl-but-2-enyl)-4-methoxymethoxy-3-methyl-5H-furan-2-one | 73,6 | 60,7 |
| Acetic acid 4-methyl-5-oxo-2-(3,7,11-trimethyl-dodeca-2,6,10-trienyl)-2,5-dihydro-furan-3-yl ester | 94 | 73 |
| Acetic acid 4-methyl-5-oxo-2-(3,7,11-trimethyl-dodeca-2,6,10-trienyl)-2,5-dihydro-furan-3-yl ester | 13,5 | 2,9 |
| Acetic acid 4-methyl-5-oxo-2-undec-10-enyl-2,5-dihydro-furan-3-yl ester | 87 | 72 |
| Acetic acid 4-methyl-5-oxo-2-(3,7,11-trimethyl-dodeca-2,6,10-trienyl)-2,5-dihydro-thiophen-3-yl ester | 37 | 25 |
| 4-Methoxymethoxy-3-methyl-5-{3-methyl-5-[3-methyl-3-(4-methyl-pent-3-enyl)-oxiranyl]-pent-2-enyl}-5H-furan-2-one | 50 | 15 |
| 5-[9-(3,3-Dimethyl-oxiranyl)-3,7-dimethyl-nona-2,6-dienyl]-4-methoxymethoxy-3-methyl-5H-furan-2-one | 76 | 42 |
| 4-Methoxy-3-methyl-5-(3,7,11-trimethyl-dodeca-2,6,10-trienyl)-5H-furan-2-one | 87 | 47 |
| 3-Methyl-4-propoxy-5-(3,7,1 1-trimethyl-dodeca-2,6,10-trienyl)-5H-furan-2-one | 51 | 12 |
| Toluene-4-sulfonic acid 4-methyl-5-oxo-2,5-dihydro-furan-3-yl ester | 60 | 65 |
| 5-(3,7-Dimethyl-octa-2,6-dienyl)-4-methoxymethoxy-3-methyl-5H-furan-2-one | 109 | 69 |
| 5-(2,2-Dimethyl-[1,3]dioxolan-4-yl)-3,4-dihydroxy-5H-furan-2-one | 78 | 72 |
| Hexadecanoic acid 2-(3,4-dihydroxy-5-oxo-2,5-dihydro-furan-2-yl)-2-hydroxy-ethyl ester | 40 | 37 |
| 3-Phenyl-acrylic acid 2-(3,4-dihydroxy-5-oxo-2,5-dihydro-furan-2-yl)-2-hydroxy-ethyl ester | 59 | 48 |
| Decanoic acid 2-(3,4-dihydroxy-5-oxo-2,5-dihydro-furan-2-yl)-2-hydroxy-ethyl ester | 24 | 14 |
| But-2-enoic acid 2-(3,4-dihydroxy-5-oxo-2,5-dihydro-furan-2-yl)-2-hydroxy-ethyl ester | 99 | 67 |
| Hexanedioic acid 2-(3,4-dihydroxy-5-oxo-2,5-dihydro-furan-2-yl)-2-hydroxy-ethyl ester vinyl ester | 95 | 58 |
| Undec-10-enoic acid 2-(3,4-dihydroxy-5-oxo-2,5-dihydro-furan-2-yl)-2-hydroxy-ethyl ester | 60 | 24 |
| Dodecanoic acid 2-(3,4-dihydroxy-5-oxo-2,5-dihydro-furan-2-yl)-2-hydroxy-ethyl ester | 19 | 9 |
| Octanoic acid 2-(3,4-dihydroxy-5-oxo-2,5-dihydro-furan-2-yl)-2-hydroxy-ethyl ester | 28 | 35 |
| Hexa-2,4-dienoic acid 2-(3,4-dihydroxy-5-oxo-2,5-dihydro-furan-2-yl)-2-hydroxy-ethyl ester | 63 | 57 |
| Chloro-acetic acid 2-(3,4-dihydroxy-5-oxo-2,5-dihydro-furan-2-yl)-2-hydroxy-ethyl ester | 90 | 69 |
| Butyric acid 2-(3,4-dihydroxy-5-oxo-2,5-dihydro-furan-2-yl)-2-hydroxy-ethyl ester | 84 | 69 |
| Tetradecanoic acid 2-(3,4-dihydroxy-5-oxo-2,5-dihydro-furan-2-yl)-2-hydroxy-ethyl ester | 7 | 5 |
| Octadecanoic acid 2-(3,4-dihydroxy-5-oxo-2,5-dihydro-furan-2-yl)-2-hydroxy-ethyl ester | 38 | 32 |
| 5-(3,7-Dimethyl-octa-2,6-dienyl)-4-hydroxy-3-methyl-5H-thiophen-2-one | 12 | 3 |
| 5-(3,7-Dimethyl-octa-2,6-dienyl)-4-hydroxy-3-methyl-5H-thiophen-2-one | - | - |
| 5-(13-Furan-3-yl-2,6,10-trimethyl-trideca-2,7,9-trienyl)-4-hydroxy-3-methyl-5H-furan-2-one | - | - |
| 5-(13-Furan-3-yl-2,6,10-trimethyl-trideca-2,7,9-trienyl)-4-hydroxy-3-methyl-5H-furan-2-one | - | - |
| 3-Methyl-4-pyrrolidin-1-yl-5-(3,7,11-trimethyl-dodeca-2,6,10-trienyl)-5H-furan-2-one | 62,17 | 39,3 |
| 4-Methyl-5-propoxy-2-(3,7,11-trimethyl-dodeca-2,6,10-trienyl)-furan-3-one | 10 | 6,73 |
| 6-(4-Methyl-5-oxo-3-pyrrolidin-1-yl-2,5-dihydro-furan-2-yl)-hexanenitrile | 78 | 67 |
| 4-(2-Methoxymethyl-pyrrolidin-1-yl)-3-methyl-5-(3,7,1 1-trimethyl-dodeca-2,6,10-trienyl)-5H-furan-2-one | 33,3 | 15 |
| 4-(2-Methoxymethyl-pyrrolidin-1-yl)-3-methyl-5-(3,7,11-trimethyl-dodeca-2,6,10-trienyl)-5H-furan-2-one | 35 | 11,4 |
| 4-sec-Butoxy-3-methyl-5-(3,7,11-trimethyl-dodeca-2,6,10-trienyl)-5H-furan-2-one | 52,65 | 8,53 |
| 5-sec-Butoxy-4-methyl-2-(3,7,11-trimethyl-dodeca-2,6,10-trienyl)-furan-3-one | 53,86 | 23,04 |
| 5-(12-Hydroxy-3,7,11-trimethyl-dodeca-2,6,10-trienyl)-3-methyl-4-pyrrolidin-1-yl-5H-furan-2-one | 91,64 | 53,09 |

## Claims

1. A compound of formula (I) 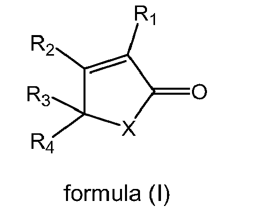 wherein
R₁ is selected from the group consisting of -H, -ORₐ, alkyl and aralkyl, wherein
Rₐ is selected from the group consisting of -H and hydroxyl protecting groups;
R₂ is selected from the group consisting of -H, -ORₐ, alkyl and -NR_{b}, wherein
Rₐ is as defined above; and
R_{b} together with the nitrogen atom forms an optionally substituted heterocyclyl group;
R₃ is -H or -O-Alkyl;
R₄ is selected from the group consisting of -H, alkyl, alkenyl, aryl, aralkyl and heterocyclyl, wherein the alkyl and the alkenyl groups are optionally substituted by at least one -ORₐ group, wherein -ORₐ has the same meaning as above;
X is an oxygen or sulphur atom; and
wherein at least two of R₁, R₂, R₃ and R₄ are not -H;
and its salts or solvates or tautomers thereof.

2. Compound according to claim 1, of formula (II) 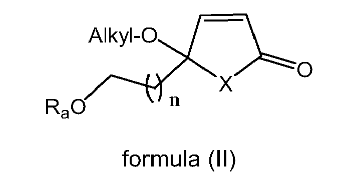 wherein
X is an oxygen or sulphur atom;
n is an integer selected from 1, 2, 3, 4, 5 and 6; and
Rₐ is defined as in claim 1;
and its salts or solvates thereof.

3. Compound according to claim 2, wherein Rₐ is a hydroxyl protecting group, preferably a silylether.

4. Compound according to claim 1, of formula (IV) 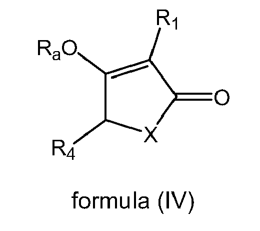 wherein
X is an oxygen or sulphur atom;
R₁, R₄ and Rₐ are as defined in claim 1;
and its salts or solvates or tautomers thereof.

5. Compound according to claim 4, wherein R₁ is methyl.

6. Compound according to any of claims 4 or 5, wherein R₄ is an alkenyl of formula (VII) 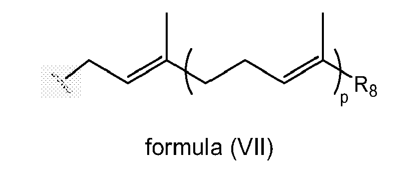 wherein
at least one double bond may be optionally replaced by an epoxide;
p is an integer selected from 0, 1, 2 or 3; and
R₈ is methyl or -ORₐ, wherein Rₐ is as defined in claim 1;
and its salts or solvates or tautomers thereof.

7. Compound according to claim 1, of formula (V) 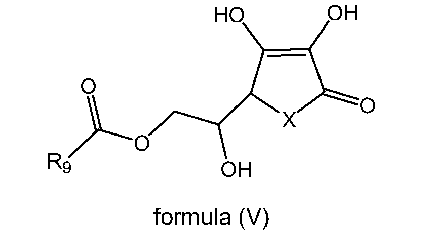 wherein
X is an oxygen or sulphur atom; and
R₉ is selected from the group consisting of -H, alkyl, alkenyl, aryl, aralkyl and heterocyclyl;
and its salts or solvates or tautomers thereof.

8. Compound according to claim 1, of formula (VI) 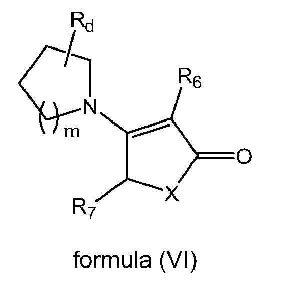 wherein
X is an oxygen or sulphur atom;
m is an integer selected from 1, 2 and 3;
R₆ is alkyl group;
R₇ is alkyl or alkenyl group; and
R_{d} is selected from the group consisting of hydrogen, substituted or unsubstituted alkyl, substituted or unsubstituted alkenyl, substituted or unsubstituted arylalkyl and substituted or unsubstituted alkoxy.
and its salts or solvates or tautomers thereof.

9. Compound according to claim 8, wherein R₇ is an alkenyl of formula (VII) 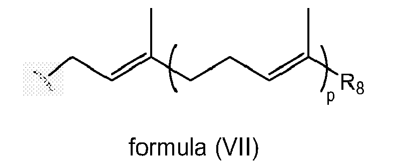 wherein
at least one double bond may be optionally replaced by an epoxide;
p is an integer selected from 0, 1, 2 and 3; and
R₈ is methyl or -ORₐ, wherein Rₐ is as defined in claim 1.

10. Compound according to claim 1, of formula (VIII) 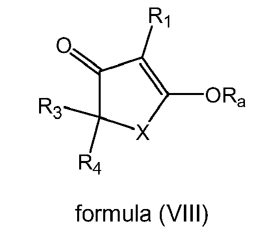 wherein X, R₁, R₃ and R₄ are defined as in claim 1; and
Rₐ is a hydroxyl protecting group;
and its salts or solvates thereof.

11. Compound according to claim 10, wherein Rₐ is an alkyl group.

12. Compound according to claim 1 selected from the group consisting of
- 5-[2-(tert-Butyl-diphenyl-silanyloxy)-ethyl]-5-methoxy-5H-furan-2-one
- 5-[3-(tert-Butyl-diphenyl-silanyloxy)-propyl]-5-methoxy-5H-furan-2-one
- 5-[4-(tert-Butyl-diphenyl-silanyloxy)-butyl]-5-methoxy-5H-furan-2-one
- 5-(4-hydroxybutyl)-5-methoxy-5H-furan-2-one
- 5-[5-(tert-Butyl-diphenyl-silanyloxy)-pentyl]-5-methoxy-5H-furan-2-one
- 5-[6-(tert-Butyl-diphenyl-silanyloxy)-hexyl]-5-methoxy-5H-furan-2-one
- 5-[4-(tert-Butyl-diphenyl-silanyloxy)-3-methyl-butyl]-4-hydroxy-3-methyl-5*H-*furan-2-one
- 5-[7-(tert-Butyl-diphenyl-silanyloxy)-2,6-dimethyl-hept-2-enyl]-4-methoxy-3-methyl-5*H*-furan-2-one
- 5-(6-Furan-3-yl-3-methyl-hex-2-enyl)-4-methoxy-3-methyl-5*H*-furan-2-one
- 4-Hydroxy-3-naphthalen-2-ylmethyl-5*H*-furan-2-one
- 3-Benzyl-4-hydroxy-5*H*-furan-2-one
- 4-Hydroxy-3-methyl-5*H*-thiophen-2-one
- 4-Hydroxy-3-pentyl-5*H*-furan-2-one
- 4-Hydroxy-3-methyl-5-(3,7,11-trimethyl-dodeca-2,6,10-trienyl)-5*H*-furan-2-one
- 4-Hydroxy-3-methyl-5-(3,7,11-trimethyl-dodeca-2,6,10-trienyl)-5*H*-thiophen-2-one
- 5-(3,7-Dimethyl-octa-2,6-dienyl)-4-hydroxy-3-methyl-5H-furan-2-one
- 4-Hydroxy-3-methyl-5-undec-10-enyl-5H-furan-2-one
- [4-(3-Hydroxy-4-methyl-5-oxo-2,5-dihydro-furan-2-ylmethyl)-phenyl]-acetic acid 2-oxo-2-phenyl-ethyl ester
- 4-Methoxymethoxy-3-methyl-5-(3,7,11-trimethyl-dodeca-2,6,10-trienyl)-5H-furan-2-one
- 5-(4-Hydroxy-3-methyl-but-2-enyl)-4-methoxymethoxy-3-methyl-5H-furan-2-one
- Acetic acid 4-methyl-5-oxo-2-(3,7,11-trimethyl-dodeca-2,6,10-trienyl)-2,5-dihydro-furan-3-yl ester
- Acetic acid 4-methyl-5-oxo-2-(3,7,11-trimethyl-dodeca-2,6,10-trienyl)-2,5-dihydro-furan-3-yl ester
- Acetic acid 4-methyl-5-oxo-2-undec-10-enyl-2,5-dihydro-furan-3-yl ester
- Acetic acid 4-methyl-5-oxo-2-(3,7,11-trimethyl-dodeca-2,6,10-trienyl)-2,5-dihydro-thiophen-3-yl ester
- 4-Methoxymethoxy-3-methyl-5-{3-methyl-5-[3-methyl-3-(4-methyl-pent-3-enyl)-oxiranyl]-pent-2-enyl}-5*H*-furan-2-one
- 5-[9-(3,3-Dimethyl-oxiranyl)-3,7-dimethyl-nona-2,6-dienyl]-4-methoxymethoxy-3-methyl-5H-furan-2-one
- 4-Methoxy-3-methyl-5-(3,7,11-trimethyl-dodeca-2,6,10-trienyl)-5*H*-furan-2-one
- 3-Methyl-4-propoxy-5-(3,7,11-trimethyl-dodeca-2,6,10-trienyl)-5*H*-furan-2-one
- Toluene-4-sulfonic acid 4-methyl-5-oxo-2,5-dihydro-furan-3-yl ester
- 5-(3,7-Dimethyl-octa-2,6-dienyl)-4-methoxymethoxy-3-methyl-5H-furan-2-one
- 5-(2,2-Dimethyl-[1,3]dioxolan-4-yl)-3,4-dihydroxy-5H-furan-2-one
- Hexadecanoic acid 2-(3,4-dihydroxy-5-oxo-2,5-dihydro-furan-2-yl)-2-hydroxy-ethyl ester
- 3-Phenyl-acrylic acid 2-(3,4-dihydroxy-5-oxo-2,5-dihydro-furan-2-yl)-2-hydroxy-ethyl ester
- Decanoic acid 2-(3,4-dihydroxy-5-oxo-2,5-dihydro-furan-2-yl)-2-hydroxy-ethyl ester
- But-2-enoic acid 2-(3,4-dihydroxy-5-oxo-2,5-dihydro-furan-2-yl)-2-hydroxy-ethyl ester
- Hexanedioic acid 2-(3,4-dihydroxy-5-oxo-2,5-dihydro-furan-2-yl)-2-hydroxy-ethyl ester vinyl ester
- Undec-10-enoic acid 2-(3,4-dihydroxy-5-oxo-2,5-dihydro-furan-2-yl)-2-hydroxy-ethyl ester
- Dodecanoic acid 2-(3,4-dihydroxy-5-oxo-2,5-dihydro-furan-2-yl)-2-hydroxy-ethyl ester
- Octanoic acid 2-(3,4-dihydroxy-5-oxo-2,5-dihydro-furan-2-yl)-2-hydroxy-ethyl ester
- Hexa-2,4-dienoic acid 2-(3,4-dihydroxy-5-oxo-2,5-dihydro-furan-2-yl)-2-hydroxy-ethyl ester
- Chloro-acetic acid 2-(3,4-dihydroxy-5-oxo-2,5-dihydro-furan-2-yl)-2-hydroxy-ethyl ester
- Butyric acid 2-(3,4-dihydroxy-5-oxo-2,5-dihydro-furan-2-yl)-2-hydroxy-ethyl ester
- Tetradecanoic acid 2-(3,4-dihydroxy-5-oxo-2,5-dihydro-furan-2-yl)-2-hydroxy-ethyl ester
- Octadecanoic acid 2-(3,4-dihydroxy-5-oxo-2,5-dihydro-furan-2-yl)-2-hydroxy-ethyl ester
- 5-(13-Furan-3-yl-2,6,10-trimethyl-trideca-2,7,9-trienyl)-4-hydroxy-3-methyl-5H-furan-2-one
- 3-Methyl-4-pyrrolidin-1-yl-5-(3,7,11-trimethyl-dodeca-2,6,10-trienyl)-5H-furan-2-one
- 4-Methyl-5-propoxy-2-(3,7,11-trimethyl-dodeca-2,6,10-trienyl)-furan-3-one
- 6-(4-Methyl-5-oxo-3-pyrrolidin-1-yl-2,5-dihydro-furan-2-yl)-hexanenitrile
- 4-(2-Methoxymethyl-pyrrolidin-1-yl)-3-methyl-5-(3,7,11-trimethyl-dodeca-2,6,10-trienyl)-5H-furan-2-one
- 4-(2-Methoxymethyl-pyrrolidin-1-yl)-3-methyl-5-(3,7,11-trimethyl-dodeca-2,6,10-trienyl)-5H-furan-2-one
- 4-sec-Butoxy-3-methyl-5-(3,7,11-trimethyl-dodeca-2,6,10-trienyl)-5H-furan-2-one
- 5-sec-Butoxy-4-methyl-2-(3,7,11-trimethyl-dodeca-2,6,10-trienyl)-furan-3-one
- 5-(12-Hydroxy-3,7,11-trimethyl-dodeca-2,6,10-trienyl)-3-methyl-4-pyrrolidin-1-yl-5H-furan-2-one
its salts or solvates or tautomers thereof.

13. Use of a compound of formula (I) 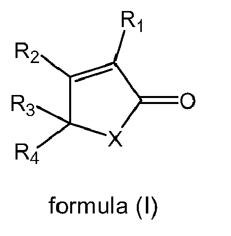 wherein
R₁ is selected from the group consisting of-H, -ORₐ, alkyl and aralkyl, wherein
Rₐ is selected from the group consisting of-H and hydroxyl protecting groups;
R₂ is selected from the group consisting of-H, -ORₐ, alkyl and -NR_{b}, wherein
Rₐ is as defined above; and
R_{b} together with the nitrogen atom forms an optionally substituted heterocyclyl group;
R₃ is -H or -O-Alkyl;
R₄ is selected from the group consisting of -H, aryl, aralkyl, heterocyclyl, alkyl and alkenyl, said alkyl or alkenyl group being optionally substituted by at least one heterocycle, preferably furane; and optionally at least one -ORa group, wherein -ORa has the same meaning as above; or
R₃ and R₄ together form an alkenyl group attached to the lactone ring by a double bond, optionally substituted by at least one heterocycle, preferably furane;
X is an oxygen or sulphur atom; and
wherein at least two of R₁, R₂, R₃ and R₄ are not -H;
and its salts or solvates or tautomers thereof;
in the manufacture of a medicament for the treatment and/or prevention of a GSK-3 mediated disease or condition.

14. Use according to claim 13, wherein the GSK-3 mediated disease or condition is selected from diabetes, conditions associated with diabetes, chronic neurodegenerative conditions including dementias such as Alzheimer's Disease, Parkinson's disease, progressive supranuclear palsy, subacute sclerosing panencephalitic parkinsonism, postencephalitic parkinsonism, pugilistic encephalitis, guam parkinsonism-dementia complex, Pick's disease, corticobasal degeneration, frontotemporal dementia, Huntington's Disease, AIDS associated dementia, amyotrophic lateral sclerosis, multiple sclerosis and neurotraumatic diseases such as acute stroke, epilepsy, mood disorders such as depression, schizophrenia and bipolar disorders, promotion of functional recovery post stroke, cerebral bleeding such as due to solitary cerebral amyloid angiopathy, hair loss, obesity, atherosclerotic cardiovascular disease, hypertension, polycystic ovary syndrome, syndrome X, ischaemia, brain injury, traumatic brain injury, cancer, leukopenia, Down's syndrome, Lewy body disease, inflammation, chronic inflammatory diseases, cancer and hyperproliferative diseases as hyperplasias and immunodeficiency.

15. Use according to claim 14, wherein the disease is Alzheimer's Disease.

16. Use according to claim 14, wherein the disease is type II diabetes.

17. Use according to claim 14, wherein the disease is depression.

18. Use according to claim 14, wherein the disease is brain injury.

19. Use according to any of claims 13 to 18, wherein said compound of formula (I) is selected from the group consisting of 5-[9-(5-Furan-3-ylmethyl-furan-3-yl)-2,6-dimethyl-non-6-enylidene]-4-hydroxy-3-methyl-5H-furan-2-one, 5-[9-(5-Furan-3-ylmethyl-furan-3-yl)-2,6-dimethyl-non-5-enylidene]-4-hydroxy-3-methyl-5H-furan-2-one and 5-(13-Furan-3-yl-2,6,10-trimethyl-trideca-6,10-dienylidene)-4-hydroxy-3-methyl-5H-furan-2-one; or its salts, solvates or tautomers thereof.

20. A compound of formula (I) as defined in any of claims 1 to 12, its salts or solvates or tautomers thereof, for use as a medicament.

21. A pharmaceutical composition comprising at least one compound of formula (I) as defined in any of claims 1 to 12, its salts or solvates or tautomers thereof, and at least one pharmaceutically acceptable carrier.

22. Use of a compound of formula (I) as defined in any of claims 1 to 12, its salts or solvates or tautomers thereof, as reagent for biological assays, preferably as a reactive for GSK-3 inhibition.

23. Method of treating or preventing a GSK-3 mediated disease or condition, which method comprises administering to a patient in need of such a treatment a therapeutically effective amount of at least one compound of formula (I) as defined in claim 13, or its salts, solvates or tautomers thereof, or a pharmaceutical composition thereof.

24. A process for the preparation of a compound according to any of claims 1-12 which comprises in any order one or more of a step selected from the group consisting of:
a) cyclation of a compound of formula (IX) in the presence of a base 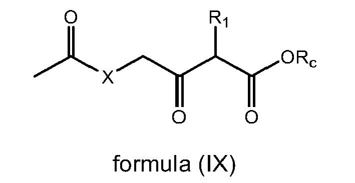 wherein R₁ and X are as defined in claim 1 and OR_{c} is selected from the group comprising substituted or unsubstituted alkyl, substituted or unsubstituted cycloalkyl, substituted or unsubstituted alkenyl, substituted or unsubstituted cycloalkenyl, substituted or unsubstituted heterocyclyl, substituted or unsubstituted aryl and substituted or unsubstituted arylalkyl;
b) bromination and further heating of a compound of formula (XI) 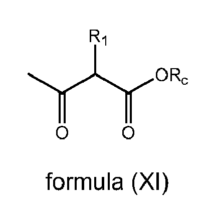 wherein R₁ is as defined in claim 1 and R_{c} is as defined above;
c) alkylation of a compound of formula (XII) 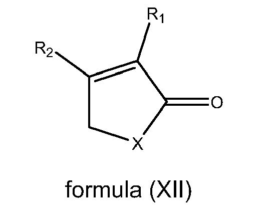 wherein R₁, R₂ and X are as defined in claim 1,
in the presence of compound of formula R₄-Hal, wherein R₄ is as defined in claim 1 and Hal is a halogen atom;
d) radical oxidation of a compound of formula (XIII) in the presence of an alcohol of formula Alkyl-OH 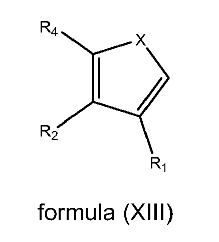 wherein R₁, R₂, R₄ and X are as defined in claim 1; and
e) esterification of ascorbic acid with a compound of formula R₉(C=O)OR_{c}, wherein R₉ is as defined in claim 7 and R_{c} is as defined above.
